# EUROPEAN PATENT SPECIFICATION

(11) **EP 1 456 369 B1**
(45) Date of publication and mention of the grant of the patent: **18.11.2009**
(21) Application number: 02782784.9
(22) Date of filing: 05.12.2002
(51) Int. Cl.: C12N 9/58, C12N 9/62

(54) **POLYPEPTIDES HAVING PROTEASE ACTIVITY AND NUCLEIC ACIDS ENCODING SAME**
PROTEASEPOLYPEPTIDE UND DAFÜR KODIERENDE POLYNUKLEOTIDE
POLYPEPTIDES A ACTIVITE PROTEASIQUE ET ACIDES NUCLEIQUES CODANT CES POLYPEPTIDES

(30) Priority: 07.12.2001 DK 200101821; 03.01.2002 DK 200200005
(43) Date of publication of application: 15.09.2004
(73) Proprietor: Novozymes A/S, 2880 Bagsvaerd (DK)
(72) Inventor: WU, Wenping, Room 103, Er Dan Yuan in Bldg No. 3, Haidian District, Beijing 100085 (CN); HATZACK, Frank, DK-2100 Copenhagen (DK); TANG, Lan, Hai Dian District, Beijing 100080 (CN)
(74) Representative: Kofoed, Gertrud Sonne
(86) International application number: PCT/DK2002/000824
(87) International publication number: WO 2003/048353

(56) References cited:
- EP-A- 1 236 405
- WO-A-99/34003
- US-A- 4 518 697
- US-A- 4 532 213
- TATSUMI H ET AL: "CLONING AND EXPRESSION IN YEAST OF A COMPLEMENTARY DNA CLONE ENCODING ASPERGILLUS-ORYZAE NEUTRAL PROTEASE II A UNIQUE METALLOPROTEASE" MOLECULAR & GENERAL GENETICS, vol. 228, no. 1-2, 1991, pages 97-103, XP002235925 ISSN: 0026-8925 -& DATABASE SWALL [Online] 1 November 1995 (1995-11-01) retrieved from EBI Database accession no. P46076 XP002902879
- DATABASE SWALL [Online] 1 November 1995 (1995-11-01) retrieved from EBI Database accession no. P47189 XP002235927 -& KOUJI MATSUMOTO ET AL: "Molecular cloning and nucleotide sequence of the complementary DNA for penicillolysin gene, plnC, an 18kDa metalloendopeptidase gene from Penicillium citrinum." BIOCHIMICA ET BIOPHYSICA ACTA, vol. 1218, 1994, pages 469-472, XP002902880 ISSN: 0167-4781
- RAMESH MATUR V ET AL: "Cloning and characterization of the cDNAs and genes (mep20) encoding homologous metalloproteinases from Aspergillus flavus and A. fumigatus." GENE (AMSTERDAM), vol. 165, no. 1, 1995, pages 121-125, XP002235964 ISSN: 0378-1119 -& DATABASE SWALL [Online] 1 November 1995 (1995-11-01) retrieved from EBI Database accession no. P46073 XP002902881 -& DATABASE SWALL [Online] 1 November 1996 (1996-11-01) retrieved from EBI Database accession no. Q09016 XP002902882
- DATABASE GSP [Online] 24 January 1994 (1994-01-24) retrieved from EBI Database accession no. AAW44369 XP002235928 -& WO 97 46689 A (GIST BROCADES B.V.) 11 December 1997 (1997-12-11)

## Description

### Background of the Invention

### Field of the invention

The present invention relates to isolated polypeptides having protease activity and isolated nucleic acid sequences encoding the polypeptides. The invention also relates to nucleic acid constructs, vectors, and host cells comprising the nucleic acid sequences as well as methods for producing and using the polypeptides.

### Description of the Related Art

The cloning of the penicillolysin gene (plnC) from a strain of *Penicillium citrinum* is disclosed by Matsumoto et al in Biochim. Biophys. Acta 1218:469 (1994). The sequence was submitted to the EMBL database as D25535.

WO 97/46689 discloses the following protease (encoding) sequences from specific *Aspergillus* strains:
SEQ ID NO:1 of WO 97/46689 is a partial *Aspergillus niger* pepH gene;
SEQ ID NO:2 of WO 97/46689 is a partial *Aspergillus niger* pepH cDNA;
SEQ ID NO:3 of WO 97/46689 is a partial *Aspergillus niger* PEPH amino acid sequence;
SEQ ID NO:4 of WO 97/46689 is an *Aspergillus nidulans* pepI gene;
SEQ ID NO:5 of WO 97/46689 is an *Aspergillus nidulans* pepI cDNA; and
SEQ ID NO:6 of WO 97/46689 is an *Aspergillus nidulans* PEPI amino acid sequence.

In Gene 165(1):121-125 (1995), Ramesh et al disclose the cloning and charaterization of the genes encoding proteases from a strain of *Aspergillus flavus* and a strain of *Aspergillus fumigatus.* These sequences were submitted to EMBL as L7524 and U24146, respectively.

The sequence of a variant neutral protease based on the neutral protease II of a strain of *Aspergillus oryzae* is disclosed in JP 05-168479. The cloning of the parent *Aspergillus oryzae neutral* protease II is described in Mol. Gen, Genet. (1991), 228, p. 97-103 by Hiroki Tatsumi et al.

The mature peptide parts of the above proteases have a degree of identity to the mature peptide part of a *Thermoascus* protease of the present invention of 75.7% or below,

The nucleotide sequences corresponding to the mature peptide parts of the above proteases have a degree of identity to the nucleotide sequence corresponding to the mature peptide part of a *Thermoascus* protease of the present invention of 68.4% or below.

WO99/34003 discloses a metalloprotease and its use in animal feed.

It is an object of the present invention to provide alternative proteases with a potential for use in animal feed.

### Summary of the Invention

The present invention relates to isolated polypeptides having protease activity selected from the group consisting of:
(a) a polypeptide having an amino acid sequence which has at least 80% identity with amino acids -178 to 177, -159 to 177, or +1 to 177 of SEQ ID NO:2;
(b) a polypeptide encoded by a nucleic acid sequence which hybridizes under low stringency conditions with
   (i) the mature protease encoding part of the plasmid contained in *Escherichia coli* DSM 14652,
   (ii) nucleotides 25 to 1089, 1 to 1089, 1 to 1344, 25 to 1344, 559 to 1344, or 559 to 1089 of SEQ ID NO:1,
   (iii) a subsequence of (i) or (ii) of at least 100 nucleotides, or
   (iv) a complementary strand of (i), (ii) or (iii);
(c) a variant of the polypeptide having an amino acid sequence of amino acids -178 to 177, -159 to 177, or +1 to 177 of SEQ ID NO:2 comprising a substitution, deletion, and/or insertion of one or more amino acids;
(d) an allelic variant of (a) or (b);
(e) a fragment of (a), (b), or (d) that has protease activity.

SEQ ID NO:1 is a cDNA sequence of a *Thermoascus aurantiacus* CGMCC No. 0670 protease (SEQ ID NO:1); and SEQ ID NO:2 is the deduced amino acid sequence thereof.

The present invention also relates to isolated nucleic acid sequences encoding the polypeptides and to nucleic acid constructs, vectors, and host cells comprising the nucleic acid sequences as well as methods for producing and using the polypeptides, in particular in animal feed.

### Brief Description of the Figures

Figure 1 shows the results of an inhibition test on a *Thermoascus aurantiacus* CGMCC No. 0670 protease;
Figure 2 shows the temperature profile of the same;
Figure 3 shows the pH-profile of the same;
Figure 4 shows the pH-stability of the same; and
Figure 5 shows the results of a substrate specificity test of the same.

### Detailed Description of the Invention

The inventors surprisingly identified a new class of proteases that may be useful in animal feed. Most of the known feed proteases are serine proteases, and many of these are not sufficiently acid-stable to survive the passage through the acidic stomach. The proteases of the invention are related to an acid-stable metalloprotease derived from *Thermoascus aurantiacus.* The mature polypeptide part of this protease comprises amino acids 1-177 of SEQ ID NO:2. *In vitro* studies simulating digestion in monogastric animals and fish showed that the *Thermoascus aurantiacus* protease is capable of increasing the amount of soluble and digestible protein and increasing the degree of protein hydrolysis. A homologous, novel polypeptide was identified in *Aspergillus oryzae* (amino acids 177-353 of SEQ ID NO:11).

### Polypeptides Having Protease Activity

Proteases are sometimes also designated peptidases, proteinases, peptide hydrolases, or proteolytic enzymes. Proteases may be of the exo-type that hydrolyses peptides starting at either end thereof, or of the endo-type that act internally in polypeptide chains (endopeptidases). Endopeptidases show activity on N- and C-terminally blocked peptide substrates that are relevant for the specificity of the protease in question.

The term "protease" is defined herein as an enzyme that hydrolyses peptide bonds. It includes any enzyme belonging to the EC 3.4 enzyme group (including each of the thirteen subclasses thereof). The EC number refers to Enzyme Nomenclature 1992 from NC-IUBMB, Academic Press, San Diego, California, including supplements 1-5 published in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650; respectively. The nomenclature is regularly supplemented and updated; see e.g. the World Wide Web (WWW) at http://www.chem.qmw.ac.uk/iubmb/enzyme/index.htm).

Proteases are classified on the basis of their catalytic mechanism into the following groups: Serine proteases (S), Cysteine proteases (C), Aspartic proteases (A), Metalloproteases (M), and Unknown, or as yet unclassified, proteases (U), see Handbook of Proteolytic Enzymes, A.J.Barrett, N.D.Rawlings, J.F.Woessner (eds), Academic Press (1998), in particular the general introduction part.

In particular embodiments, the proteases of the invention are selected from the group consisting of:
(a) proteases belonging to the EC 3.4.24 metalloendopeptidases;
(b) metalloproteases belonging to the M group of the above Handbook;
(c) metalloproteases not yet assigned to clans (designation: Clan MX), or belonging to either one of clans MA, MB, MC, MD, ME, MF, MG, MH (as defined at pp. 989-991 of the above Handbook);
(d) other families of metalloproteases (as defined at pp. 1448-1452 of the above Handbook);
(e) metalloproteases with a HEXXH motif;
(f) metalloproteases with an HEFTH motif;
(g) metalloproteases belonging to either one of families M3, M26, M27, M32, M34, M35, M36, M41, M43, or M47 (as defined at pp. 1448-1452 of the above Handbook); and
(h) metalloproteases belonging to family M35 (as defined at pp. 1492-1495 of the above Handbook).

In other particular embodiments, metalloproteases are hydrolases in which the nucleophilic attack on a peptide bond is mediated by a water molecule, the water molecule being activated by a divalent metal cation. Examples of divalent cations are zinc, cobalt or manganese. The metal ion may be held in place by amino acid ligands, The number of ligands may be five, four, three, two, one or zero. In a particular embodiment the number is two or three, preferably three.

For determining whether a given protease is a metalloprotease or not, reference is made to the above Handbook and the principles indicated therein. Such determination can be carried out for all types of proteases, be it naturally occurring or wild-type proteases; or genetically engineered or synthetic proteases.

Protease activity can be measured using any assay, in which a substrate is employed, that includes peptide bonds relevant for the specificity of the protease in question. Assay-pH and assay-temperature are likewise to be adapted to the protease in question. Examples of assay-pH-values are pH 6, 7, 8, 9, 10, or 11. Examples of assay-temperatures are 30, 35, 37, 40, 45, 50, 55, 60, 65, 70 or 80°C.

Examples of protease substrates are casein, such as Azurine-Crosslinked Casein (AZCL-casein). Two protease assays are described in Example 1 herein, of which the so-called AZCL-Casein Assay is a preferred assay for the purposes of the present invention.

There are no limitations on the origin of the protease according to the invention. Thus, the term protease includes not only natural or wild-type proteases obtained from microorganisms of any genus, but also any mutants, variants, fragments etc. thereof exhibiting protease activity, as well as synthetic proteases, such as shuffled proteases, and consensus proteases. Such genetically engineered proteases can be prepared as is generally known in the art, eg by Site-directed Mutagenesis, by PCR (using a PCR fragment containing the desired mutation as one of the primers in the PCR reactions), or by Random Mutagenesis. The preparation of consensus proteins is described in eg EP 897985. The term "obtained from" as used herein in connection with a given source shall mean that the polypeptide encoded by the nucleic acid sequence is produced by the source or by a cell in which the nucleic acid sequence from the source is present. In a preferred embodiment, the polypeptide is secreted extracellularly.

In a specific embodiment, the protease is a low-allergenic variant, designed to invoke a reduced immunological response when exposed to animals, including man. The term immunological response is to be understood as any reaction by the immune system of an animal exposed to the protease. One type of immunological response is an allergic response leading to increased levels of IgE in the exposed animal. Low-allergenic variants may be prepared using techniques known in the art. For example the protease may be conjugated with polymer moieties shielding portions or epitopes of the protease involved in an immunological response. Conjugation with polymers may involve *in vitro* chemical coupling of polymer to the protease, e.g. as described in WO 96/17929, WO98/30682, WO98/35026, and/or WO99/00489. Conjugation may in addition or alternatively thereto involve in vivo coupling of polymers to the protease. Such conjugation may be achieved by genetic engineering of the nucleotide sequence encoding the protease, inserting consensus sequences encoding additional glycosylation sites in the protease and expressing the protease in a host capable of glycosylating the protease, see e.g. WO00/26354. Another way of providing low-allergenic variants is genetic engineering of the nucleotide sequence encoding the protease so as to cause the protease to self-oligomerize, effecting that protease monomers may shield the epitopes of other protease monomers and thereby lowering the antigenicity of the oligomers. Such products and their preparation is described e.g. in WO96/16177. Epitopes involved in an immunological response may be identified by various methods such as the phage display method described in WO00/26230 and WO 01/83559, or the random approach described in EP 561907. Once an epitope has been identified, its amino acid sequence may be altered to produce altered immunological properties of the protease by known gene manipulation techniques such as site directed mutagenesis (see e.g. WO 00/26230, WO 00/26354 and/or WO00/22103) and/or conjugation of a polymer may be done in sufficient proximity to the epitope for the polymer to shield the epitope.

The present invention relates to isolated polypeptides comprising an amino acid sequence which has a degree of identity to amino acids -178 to 177, -159 to 177, or preferably amino acids 1 to 177 (the mature polypeptide) of SEQ ID NO:2 of at least about 64%, or at least about 65%, or at least about 70%, or at least about 75%, or at least about 76%, or at least about 77%, or at least about 78%, or at least about 79%, or at least about 80%, or at least about 82%, or at least about 85%, or at least about 90%, or at least about 95%, or at least about 97%; and which have protease activity (hereinafter "homologous polypeptides"). In particular embodiments, the polypeptides of the invention i) have; or ii) consist of an amino acid sequence with a degree of identity as mentioned above.

The *Thermoascus aurantiacus* protease, the mature polypeptide of which comprises amino acids 1-177 of SEQ ID NO:2 is of course one example of a polypeptide of the invention.

Herein described is another polypeptide derived from *Aspergillus oryzae* and comprises SEQ ID NO:11, or amino acids -23-353; -23-374; -23-397; 1-353; 1-374; 1-397; 177-353; 177-374; or 177-397 thereof, and is encoded by SEQ ID NO:10, or nucleotides 2-1129; 2-1195; 2-1267; 71-1129; 71-1195; 71-1267, 599-1129; 599-1195; or 599-1267 thereof, respectively.

For purposes of the present invention the degree of identity between two amino acid sequences, as well as the degree of identity between two nucleotide sequences, is determined by the program "align" which is a Needleman-Wunsch alignment (i.e. a global alignment). The program is used for alignment of polypeptide, as well as nucleotide sequences. The default scoring matrix BLOSUM50 is used for polypeptide alignments, and the default identity matrix is used for nucleotide alignments. The penalty for the first residue of a gap is -12 for polypeptides and -16 for nucleotides. The penalties for further residues of a gap are -2 for polypeptides, and -4 for nucleotides.

"Align" is part of the FASTA package version v20u6 (see W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA," Methods in Enzymology 183:63- 98). FASTA protein alignments use the Smith-Waterman algorithm with no limitation on gap size (see "Smith-Waterman algorithm", T. F. Smith and M. S. Waterman (1981) J. Mol. Biol. 147:195-197).

In a particular embodiment, the homologous polypeptides have an amino acid sequence that differs by fourty, thirtyfive, thirty, twentyfive, twenty, or by fifteen amino acids. In another embodiment, the homologous polypeptides have an amino acid sequence that differs by ten, or by nine, or by eight, or by seven, or by six, or by five amino acids. In another particular embodiment, the homologous polypeptides differ by four, or by three, or by two amino acids, or by one amino acid from amino acids -178 to 177, -159 to 177, or +1 to 177 of SEQ ID NO:2.

In particular embodiments, the polypeptides of the present invention a) comprise, or b) consist of
i) the amino acid sequence of amino acids -178 to 177, -159 to 177, or +1 to 177 of SEQ ID NO:2;
ii) the amino acid sequence of amino acids -23-353, -23-374, -23-397, 1-353, 1-374, 1-397, 177-353, 177-374, or 177-397 of SEQ ID NO:11; or
allelic variants, or fragments, of the sequences of i) and ii) that have protease activity.

A fragment of amino acids -178 to 177, -159 to 177, or +1 to 177 of SEQ ID NO:2 or of amino acids -23-353, -23-374, -23-397, 1-353, 1-374, 1-397, 177-353, 177-374, or 177-397 of SEQ ID NO:11; is a polypeptide having one or more amino acids deleted from the amino and/or carboxyl terminus of these amino acid sequences. In one embodiment a fragment contains at least 75 amino acid residues, or at least 100 amino acid residues, or at least 125 amino acid residues, or at least 150 amino acid residues, or at least 160 amino acid residues, or at least 165 amino acid residues, or at least 170 amino acid residues, or at least 175 amino acid residues.

An allelic variant denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

The present invention also relates to isolated polypeptides having protease activity and which are encoded by nucleic acid sequences which hybridize under very low, or low, or medium, or medium-high, or high, or very high stringency conditions with a nucleic acid probe which hybridizes under the same conditions with (a) nucleotides 25 to 1089, 1 to 1089, 1 to 1344, 25 to 1344, 559 to 1344, or preferably nucleotides 559 to 1089 of SEQ ID NO:1, (b) the cDNA sequence contained in nucleotides 559 to 1089 of SEQ ID NO:1, (c) a subsequence of (a) or (b), or (d) a complementary strand of (a), (b), or (c) (J. Sambrook, E.F. Fritsch, and T. Maniatis, 1989, *Molecular Cloning, A Laboratory Manual,* 2nd edition, Cold Spring Harbor, New York). In one particular embodiment the nucleic acid probe is selected from amongst the nucleic acid sequences of (a), (b), (c) or (d) above.

The subsequence of nucleotides 559 to 1089, 25 to 1089, 1 to 1089, 1 to 1344, 25 to 1344, or 559 to 1344 of SEQ ID NO:1 may be at least 100 nucleotides, or in another embodiment at least 200 nucleotides. Moreover, the subsequence may encode a polypeptide fragment that has protease activity.

The nucleic acid sequences of nucleotides 559 to 1089, 25 to 1089, 1 to 1089, 1 to 1344, 25 to 1344, or 559 to 1344 of SEQ ID NO:1 or a subsequence thereof, as well as the amino acid sequences of amino acids -178 to 177, -159 to 177, or +1 to 177 of SEQ ID NO:2 or a fragment thereof, may be used to design a nucleic acid probe to identify and clone DNA encoding polypeptides having protease activity from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 35 nucleotides in length. Longer probes can also be used. Both DNA and RNA probes can be used. The probes are typically labeled for detecting the corresponding gene (for example, with ³²P, ³H, ³⁵S, biotin, or avidin). Such probes are encompassed by the present invention.

Thus, a genomic DNA or cDNA library prepared from such other organisms may be screened for DNA that hybridizes with the probes described above and which encodes a polypeptide having protease activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA which is homologous with SEQ ID NO:1 or a subsequence thereof, the carrier material is used in a Southern blot. For purposes of the present invention, hybridization indicates that the nucleic acid sequence hybridizes to a labeled nucleic acid probe corresponding to the nucleic acid sequence shown in SEQ ID NO:1, its complementary strand, or a subsequence thereof, under very low to very high stringency conditions. Molecules to which the nucleic acid probe hybridizes under these conditions are detected using X-ray film.

In a particular embodiment, the nucleic acid probe is a nucleic acid sequence which encodes amino acids -178 to 177, -159 to 177, or +1 to 177 of SEQ ID NO:2, or subsequences thereof. In another embodiment, the nucleic acid probe is nucleotides 25 to 1089, 1 to 1089, 1 to 1344, 25 to 1344, 559 to 1344, or preferably nucleotides 559 to 1089 of SEQ ID NO:1 (the mature polypeptide coding region of SEQ ID NO:1). In another preferred embodiment, the nucleic acid probe is the nucleic acid sequence, or preferably the mature polypeptide coding region thereof, which is contained in the plasmid which is contained in *Escherichia coli* DSM 14652, wherein the nucleic acid sequence encodes a polypeptide having protease activity.

For long probes of at least 100 nucleotides in length, very low to very high stringency conditions are defined as prehybridization and hybridization at 42°C in 5X SSPE, 0.3% SDS, 200 µg/ml sheared and denatured salmon sperm DNA, and either 25% formamide for very low and low stringencies, 35% formamide for medium and medium-high stringencies, or 50% formamide for high and very high stringencies, following standard Southern blotting procedures.

For long probes of at least 100 nucleotides in length, the carrier material is finally washed three times each for 15 minutes using 2 x SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For short probes about 15 nucleotides to about 70 nucleotides in length, stringency conditions are defined as prehybridization, hybridization, and washing post-hybridization at 5°C to 10°C below the calculated Tₘ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes about 15 nucleotides to about 70 nucleotides in length, the carrier material is washed once in 6X SCC plus 0.1% SDS for 15 minutes and twice each for 15 minutes using 6X SSC at 5°C to 10°C below the calculated Tₘ.

The present invention also relates to variants of the polypeptide having an amino acid sequence of amino acids 1 to 177, -159 to 177, or -178 to 177 of SEQ ID NO:2 comprising a substitution, deletion, and/or insertion of one or more amino acids.

The amino acid sequences of the variant polypeptides may differ from the amino acid sequence of amino acids 1 to 177, -159 to 177, or -178 to 177 of SEQ ID NO:2 by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter the specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

The present invention also relates to isolated polypeptides having protease activity, and selected from the group consisting of the following:
(f) polypeptides having
   (i) a calculated molecular weight of about 17 to about 22kDa, preferably of about 18 to about 21kDa, or from about 19 to about 20kDa; or an experimental molecular weight (by SDS-PAGE) of about 19 to about 27kDa, preferably of about 20 to about 26kDa, or of about 21 to about 25kDa, or of about 22 to about 24kDa,
   (ii) a pl of about pH7 to about pH10, preferably of about pH7.5 to about pH9.5, or from about pH8.0 to about pH 9.0,
   (iii) an optimum pH of about pH5.5 to about pH8.0, or preferably of about pH5.5 to about pH7.0, or of about pH5.5 to about pH6.5, using the AZCL-casein assay at 45°C and the succinic acid buffer system, and/or
   (iv) an optimum temperature of about 45 to about 90°C, preferably of about 50 to about 85°C, or of about 60 to about 80°C, using the AZCL-casein assay at pH9;
(g) polypeptides which are not inhibited by
   (v) SSI (the Streptomyces Subtilisin Inhibitor), and/or not inhibited by
   (vi) EDTA,
   using the AZCL-casein assay at pH9 and 45°C;
(h) polypeptides which are stable in the range of about pH 3.5 to about pH10.5, or preferably in the range of about pH3.5 to about pH9.0, or in the range of about pH4.0 to pH5.0, after 2 hours incubation at 37°C in the succinic acid buffer system, the residual activity being measured using the AZCL-casein assay at pH9 and 45°C;
(k) polypeptides which are synthesized with an N-terminal propeptide; and
(I) polypeptides having an HEXXH motif.

Polypeptide stability as referred to in (h) above means that the residual activity is at least 40%, or 50%, or 60%, or 70%, or 80% as compared to a control sample that has not been pre-incubated for 2 hours. Polypeptides that are stable according to this definition may be designated acid-stable polypeptides.

A polypeptide of the present invention may be a bacterial polypeptide. For example, the polypeptide may be a gram positive bacterial polypeptide such as a *Bacillus* polypeptide, or a *Streptomyces* polypeptide; or a gram negative bacterial polypeptide, *e.g.,* an *E*. *coli* or a *Pseudomonas* sp. polypeptide.

A polypeptide of the present invention may be a fungal polypeptide, and more preferably a yeast polypeptide such as a *Candida, Kluyveromyces, Pichia, Saccharomyces, Schizosaccharomyces,* or *Yarrowia* polypeptide; or more preferably a filamentous fungal polypeptide such as an *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium,* or *Trichoderma* polypeptide.

In another embodiment, the polypeptide is an *Aspergillus aculeatus, Aspergillus awamori, Aspergillus foetidus, Aspergillus japonicus, Aspergillus nidulans, Aspergillus niger, Aspergillus oryzae, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Thermoascus aurantiacus, Trichoderma harzianum, Trichoderma koningii, Trichoderma longibrachiatum, Trichoderma reesei,* or *Trichoderma viride* polypeptide.

In another embodiment, the polypeptide is a polypeptide derived from a filamentous fungus of the phylum *Ascomycota,* preferably of the class *Pezizomycotina,* more preferably of the order *Eurotiomycetes,* even more preferably of the sub-family *Eurotiales,* and most preferably of the family *Trichocomaceae.*

In still another embodiment, the polypeptide is derived from a fungus of the genus *Thermoascus,* for example the species *Thermoascus aurantiacus,* such as the strain *Thermoascus aurantiacus* CGMCC No. 0670, *e.g*., a polypeptide with the amino acid sequence of amino acids -178 to 177, -159 to 177, or +1 to 177 of SEQ ID NO:2.

It will be understood that for the aforementioned species, the invention encompasses both the perfect and imperfect states, and other taxonomic equivalents, *e.g*., anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Furthermore, such polypeptides may be identified and obtained from other sources including microorganisms isolated from nature (*e.g*., soil, composts, water, etc.) using the above-mentioned probes. Techniques for isolating microorganisms from natural habitats are well known in the art. The nucleic acid sequence may then be derived by similarly screening a genomic or cDNA library of another microorganism. Once a nucleic acid sequence encoding a polypeptide has been detected with the probe(s), the sequence may be isolated or cloned by utilizing techniques which are known to those of ordinary skill in the art (see, *e.g.,* Sambrook *et al.,* 1989, *supra).*

As defined herein, an "isolated" polypeptide is a polypeptide which is essentially free of other non-protease polypeptides, *e.g*., at least about 20% pure, preferably at least about 40% pure, more preferably about 60% pure, even more preferably about 80% pure, most preferably about 90% pure, and even most preferably about 95% pure, as determined by SDS-PAGE.

Polypeptides encoded by nucleic acid sequences of the present invention also include fused polypeptides or cleavable fusion polypeptides in which another polypeptide is fused at the N-terminus or the C-terminus of the polypeptide or fragment thereof. A fused polypeptide is produced by fusing a nucleic acid sequence (or a portion thereof) encoding another polypeptide to a nucleic acid sequence (or a portion thereof) of the present invention. Techniques for producing fusion polypeptides are known in the art, and include ligating the coding sequences encoding the polypeptides so that they are in frame and that expression of the fused polypeptide is under control of the same promoter(s) and terminator.

### Nucleic Acid Sequences

The present invention also relates to isolated nucleic acid sequences that encode a polypeptide of the present invention. Particular nucleic acid sequences of the invention are nucleotides 25 to 1089, 1 to 1089, 1 to 1344, 25 to 1344, 559 to 1344, and in particular nucleotides 559 to 1089 of SEQ ID NO:1, the latter corresponding to the mature polypeptide encoding region. Another particular nucleic acid sequence of the invention is the sequence, preferably the mature polypeptide encoding region thereof, which is contained in the plasmid that is contained in the deposited microorganism *Escherichia coli* DSM 14652. The present invention also encompasses nucleic acid sequences which encode a polypeptide having the amino acid sequence of amino acids 1 to 177, -159 to 177, or -178 to 177 of SEQ ID NO:2, which differ from the corresponding parts of SEQ ID NO:1 by virtue of the degeneracy of the genetic code. The present invention also relates to subsequences of SEQ ID NO:1 which encode fragments of SEQ ID NO:2 that have protease activity.

The present invention also relates to isolated nucleic acid sequences that encode a polypeptide of the present invention. Particular nucleic acid sequences of the invention are SEQ ID NO:10, or nucleotides 2-1129; 2-1195; 2-1267; 71-1129; 71-1195; 71-1267; 599-1129; 599-1195; or 599-1267 thereof, nucleotides 71-1129 of SEQ ID NO:10 corresponding to the mature polypeptide encoding region.

A subsequence of SEQ ID NO:1 is a nucleic acid sequence encompassed by SEQ ID NO:1 except that one or more nucleotides from the 5' and/or 3' end have been deleted. Preferably, a subsequence contains at least 225 nucleotides, more preferably at least 300 nucleotides, even more preferably at least 375, 450, 500, 531, 600, 700, 800, 900, 1000, 1100, 1200, or 1300 nucleotides.

The present invention also relates to nucleotide sequences which have a degree of identity to nucleotides 25 to 1089, 1 to 1089, 1 to 1344, 25 to 1344, 559 to 1344, or preferably nucleotides 559 to 1089 of SEQ ID NO:1 of at least 69, 70, 72, 74, 76 or 80%. In a particular embodiment the degree of identity is at least 85%, at least 90%, at least 95%, or at least 97%. For determining the degree of nucleotide identity, the program "align" is used which is referred to above.

In a particular embodiment the invention relates to nucleotide sequences which have a degree of identity to nucleotides 25 to 1089, 1 to 1089, 1 to 1344, or 25 to 1344 of SEQ ID NO:1 of at least 55, 60, 62, 64, 66, 68, 70, 72, 74, 76 or 80%. In a particular embodiment the degree of identity is at least 85%, at least 90%, at least 95%, or at least 97%. For determining the degree of nucleotide identity, the program "align" is used which is referred to above.

The present invention also relates to mutant nucleic acid sequences comprising at least one mutation in nucleotides 25 to 1089, 1 to 1089, 1 to 1344, 25 to 1344, 559 to 1344, or 559 to 1089 of SEQ ID NO:1, in which the mutant nucleic acid sequence encodes a polypeptide which (i) consists of amino acids -178 to 177, -159 to 177, or +1 to 177 of SEQ ID NO:2, or (ii) is a variant of any of the sequences of (i), wherein the variant comprises a substitution, deletion, and/or insertion of one or more amino acids, or (iii) is an allelic variant of any of the sequences of (i), or (iv) is a fragment of any of the sequences of (i).

The techniques used to isolate or clone a nucleic acid sequence encoding a polypeptide are known in the art and include isolation from genomic DNA, preparation from cDNA, or a combination thereof. The cloning of the nucleic acid sequences of the present invention from such genomic DNA can be effected, *e.g*., by using the well known polymerase chain reaction (PCR) or antibody screening of expression libraries to detect cloned DNA fragments with shared structural features. See, *e.g*., Innis et al., 1990, PCR: A Guide to Methods and Application, Academic Press, New York. Other nucleic acid amplification procedures such as ligase chain reaction (LCR), ligated activated transcription (LAT) and nucleic acid sequence-based amplification (NASBA) may be used. The nucleic acid sequence may be cloned from a strain of *Thermoascus,* or another or related organism and thus, for example, may be an allelic or species variant of the polypeptide encoding region of the nucleic acid sequence.

The term "isolated nucleic acid sequence" as used herein refers to a nucleic acid sequence which is essentially free of other nucleic acid sequences, *e.g*., at least about 20% pure, preferably at least about 40% pure, more preferably at least about 60% pure, even more preferably at least about 80% pure, and most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

The present invention also relates to nucleic acid sequences which have a degree of homology to the mature polypeptide coding sequence of SEQ ID NO:1 (*i.e*., nucleotides 559 to 1089) of at least about 70%, preferably about 75%, preferably about 80%, more preferably about 90%, even more preferably about 95%, and most preferably about 97% homology, which encode an active polypeptide. For purposes of the present invention, the degree of homology between two nucleic acid sequences is determined by the "align" program described above.

Modification of a nucleic acid sequence encoding a polypeptide of the present invention may be necessary for the synthesis of polypeptides substantially similar to the polypeptide. The term "substantially similar" to the polypeptide refers to non-naturally occurring forms of the polypeptide. These polypeptides may differ in some engineered way from the polypeptide isolated from its native source, *e.g*., variants that differ in specific activity, thermostability, pH optimum, or the like. The variant sequence may be constructed on the basis of the nucleic acid sequence presented as the polypeptide encoding part of SEQ ID NO:1, *e.g*., a subsequence thereof, and/or by introduction of nucleotide substitutions which do not give rise to another amino acid sequence of the polypeptide encoded by the nucleic acid sequence, but which correspond to the codon usage of the host organism intended for production of the protease, or by introduction of nucleotide substitutions which may give rise to a different amino acid sequence. For a general description of nucleotide substitution, see, *e.g*., Ford et al., 1991, Protein Expression and Purification 2: 95-107.

It will be apparent to those skilled in the art that such substitutions can be made outside the regions critical to the function of the molecule and still result in an active polypeptide. Amino acid residues essential to the activity of the polypeptide encoded by the isolated nucleic acid sequence of the invention, and therefore preferably not subject to substitution, may be identified according to procedures known in the art, such as site-directed mutagenesis or alanine-scanning mutagenesis (see, *e.g*., Cunningham and Wells, 1989, Science 244: 1081-1085). In the latter technique, mutations are introduced at every positively charged residue in the molecule, and the resultant mutant molecules are tested for protease activity to identify amino acid residues that are critical to the activity of the molecule. Sites of substrate-protease interaction can also be determined by analysis of the three-dimensional structure as determined by such techniques as nuclear magnetic resonance analysis, crystallography or photoaffinity labelling (see, *e.g*., de Vos et al., 1992, Science 255: 306-312; Smith et al., 1992, Journal of Molecular Biology 224: 899-904; Wlodaver et al., 1992, FEBS Letters 309: 59-64).

The present invention also relates to isolated nucleic acid sequences encoding a polypeptide of the present invention, which hybridize under very low stringency conditions, preferably low stringency conditions, more preferably medium stringency conditions, more preferably medium-high stringency conditions, even more preferably high stringency conditions, and most preferably very high stringency conditions with a nucleic acid probe which hybridizes under the same conditions with the nucleic acid sequence of SEQ ID NO:1 or its complementary strand; or allelic variants and subsequences thereof (Sambrook *et al.,* 1989, *supra),* as defined herein.

The present invention also relates to isolated nucleic acid sequences produced by (a) hybridizing a DNA under very low, low, medium, medium-high, high, or very high stringency conditions with (i) nucleotides nucleotides 559 to 1089, 25 to 1089, 1 to 1089, 1 to 1344, 25 to 1344, or 559 to 1344 of SEQ ID NO:1, (ii) the cDNA sequence contained in nucleotides nucleotides 559 to 1089, 25 to 1089, 1 to 1089, 1 to 1344, 25 to 1344, or 559 to 1344 of SEQ ID NO:1, (iii) a subsequence of (i) or (ii), or (iv) a complementary strand of (i), (ii), or (iii); and (b) isolating the nucleic acid sequence. The subsequence is preferably a sequence of at least 100 nucleotides such as a sequence that encodes a polypeptide fragment which has protease activity.

### Methods for Producing Mutant Nucleic Acid Sequences

The present invention further relates to methods for producing a mutant nucleic acid sequence, comprising introducing at least one mutation into the mature polypeptide coding sequence of SEQ ID NO:1 or a subsequence thereof, wherein the mutant nucleic acid sequence encodes a polypeptide which consists of amino acids 1 to 177, -159 to 177, or -178 to 177 of SEQ ID NO:2; or a fragment thereof which has protease activity.

The introduction of a mutation into the nucleic acid sequence to exchange one nucleotide for another nucleotide may be accomplished by site-directed mutagenesis using any of the methods known in the art. Particularly useful is the procedure that utilizes a supercoiled, double stranded DNA vector with an insert of interest and two synthetic primers containing the desired mutation. The oligonucleotide primers, each complementary to opposite strands of the vector, extend during temperature cycling by means of *Pfu* DNA polymerase. On incorporation of the primers, a mutated plasmid containing staggered nicks is generated. Following temperature cycling, the product is treated with *Dpn*I which is specific for methylated and hemimethylated DNA to digest the parental DNA template and to select for mutation-containing synthesized DNA. Other procedures known in the art may also be used.

### Nucleic Acid Constructs

The present invention also relates to nucleic acid constructs comprising a nucleic acid sequence of the present invention operably linked to one or more control sequences that direct the expression of the coding sequence in a suitable host cell under conditions compatible with the control sequences. Expression will be understood to include any step involved in the production of the polypeptide including, but not limited to, transcription, post-transcriptional modification, translation, post-translational modification, and secretion.

"Nucleic acid construct" is defined herein as a nucleic acid molecule, either single- or double-stranded, which is isolated from a naturally occurring gene or which has been modified to contain segments of nucleic acid combined and juxtaposed in a manner that would not otherwise exist in nature. The term nucleic acid construct is synonymous with the term expression cassette when the nucleic acid construct contains all the control sequences required for expression of a coding sequence of the present invention. The term "coding sequence" is defined herein as a nucleic acid sequence that directly specifies the amino acid sequence of its protein product. The boundaries of the coding sequence are generally determined by a ribosome binding site (prokaryotes) or by the ATG start codon (eukaryotes) located just upstream of the open reading frame at the 5' end of the mRNA and a transcription terminator sequence located just downstream of the open reading frame at the 3' end of the mRNA. A coding sequence can include, but is not limited to, DNA, cDNA, and recombinant nucleic acid sequences.

An isolated nucleic acid sequence encoding a polypeptide of the present invention may be manipulated in a variety of ways to provide for expression of the polypeptide. Manipulation of the nucleic acid sequence prior to its insertion into a vector may be desirable or necessary depending on the expression vector. The techniques for modifying nucleic acid sequences utilizing recombinant DNA methods are well known in the art.

The term "control sequences" is defined herein to include all components that are necessary or advantageous for the expression of a polypeptide of the present invention. Each control sequence may be native or foreign to the nucleic acid sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide. The term "operably linked" is defined herein as a configuration in which a control sequence is appropriately placed at a position relative to the coding sequence of the DNA sequence such that the control sequence directs the expression of a polypeptide.

The control sequence may be an appropriate promoter sequence, a nucleic acid sequence that is recognized by a host cell for expression of the nucleic acid sequence. The promoter sequence contains transcriptional control sequences that mediate the expression of the polypeptide. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase *(glaA), Rhizomucor miehei* lipase, *Aspergillus oryzae* alkaline protease, *Aspergillus oryzae* triose phosphate isomerase, *Aspergillus nidulans* acetamidase, and *Fusarium oxysporum* trypsin-like protease (WO 96/00787), as well as the NA2-tpi promoter (a hybrid of the promoters from the genes for *Aspergillus niger* neutral alpha-amylase and *Aspergillus oryzae* triose phosphate isomerase), and mutant, truncated, and hybrid promoters thereof.

The control sequence may also be a suitable transcription terminator sequence, a sequence recognized by a host cell to terminate transcription. The terminator sequence is operably linked to the 3' terminus of the nucleic acid sequence encoding the polypeptide. Any terminator which is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

The control sequence may also be a suitable leader sequence, a nontranslated region of an mRNA which is important for translation by the host cell. The leader sequence is operably linked to the 5' terminus of the nucleic acid sequence encoding the polypeptide. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

The control sequence may also be a polyadenylation sequence, a sequence operably linked to the 3' terminus of the nucleic acid sequence and which, when transcribed, is recognized by the host cell as a signal to add polyadenosine residues to transcribed mRNA. Any polyadenylation sequence which is functional in the host cell of choice may be used in the present invention.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

The control sequence may also be a signal peptide coding region that codes for an amino acid sequence linked to the amino terminus of a polypeptide and directs the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for filamentous fungal host cells are the signal peptide coding regions obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, and *Humicola lanuginosa* lipase.

In a preferred embodiment, the signal peptide coding region is nucleotides 25 to 81 of SEQ ID NO:1 which encode amino acids 1 to 19 of SEQ ID NO:2.

The control sequence may also be a propeptide coding region that codes for an amino acid sequence positioned at the amino terminus of a polypeptide. The resultant polypeptide is known as a proprotease or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (aprE), *Bacillus subtilis* neutral protease *(nprT), Saccharomyces cerevisiae* alpha-factor, *Rhizomucor miehei* aspartic proteinase, and *Myceliophthora thermophila* laccase (WO 95/33836).

In a preferred embodiment, the propeptide coding region is nucleotides 82 to 558 of SEQ ID NO:1 which encode amino acids 20 to 178 of SEQ ID NO:2.

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the nucleic acid sequence encoding the polypeptide would be operably linked with the regulatory sequence.

### Expression Vectors

The present invention also relates to recombinant expression vectors comprising a nucleic acid sequence of the present invention, a promoter, and transcriptional and translational stop signals. The various nucleic acid and control sequences described above may be joined together to produce a recombinant expression vector which may include one or more convenient restriction sites to allow for insertion or substitution of the nucleic acid sequence encoding the polypeptide at such sites. Alternatively, the nucleic acid sequence of the present invention may be expressed by inserting the nucleic acid sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (e.g., a plasmid or virus) which can be conveniently subjected to recombinant DNA procedures and can bring about the expression of the nucleic acid sequence. The choice of the vector will typically depend on the compatibility of the vector with the host cell into which the vector is to be introduced. The vectors may be linear or closed circular plasmids.

The vector may be an autonomously replicating vector, *i.e*., a vector which exists as an extrachromosomal entity, the replication of which is independent of chromosomal replication, *e.g*., a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the *dal* genes from *Bacillus subtilis* or *Bacillus licheniformis,* or markers which confer antibiotic resistance such as ampicillin, kanamycin, chloramphenicol or tetracycline resistance. Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), sC (sulfate adenyltransferase), *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*

The vectors of the present invention preferably contain an element(s) that permits stable integration of the vector into the host cell's genome or autonomous replication of the vector in the cell independent of the genome.

For integration into the host cell genome, the vector may rely on the nucleic acid sequence encoding the polypeptide or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleic acid sequences. On the other hand, the vector may be integrated into the genome of the host cell by non-homologous recombination.

For autonomous replication, the vector may further comprise an origin of replication enabling the vector to replicate autonomously in the host cell in question. Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.* Examples of origins of replication for use in a yeast host cell are the 2 micron origin of replication, ARS1, ARS4, the combination of ARS1 and CEN3, and the combination of ARS4 and CEN6. The origin of replication may be one having a mutation which makes its functioning temperature-sensitive in the host cell (see, *e.g*., Ehrlich, 1978, Proceedings of the National Academy of Sciences USA 75: 1433).

More than one copy of a nucleic acid sequence of the present invention may be inserted into the host cell to increase production of the gene product. An increase in the copy number of the nucleic acid sequence can be obtained by integrating at least one additional copy of the sequence into the host cell genome or by including an amplifiable selectable marker gene with the nucleic acid sequence where cells containing amplified copies of the selectable marker gene, and thereby additional copies of the nucleic acid sequence, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra).*

The protease of the invention may also be co-expressed together with at least one other enzyme of interest for animal feed, such as phytase, xylanase, galactanase, and/or beta-glucanase. The enzymes may be co-expressed from different vectors, from one vector, or using a mixture of both techniques. When using different vectors, the vectors may have different selectable markers, and different origins of replication. When using only one vector, the genes can be expressed from one or more promoters. If cloned under the regulation of one promoter (di- or multi-cistronic), the order in which the genes are cloned may affect the expression levels of the proteins. The protease may also be expressed as a fusion protein, i.e. that the gene encoding the protease has been fused in frame to the gene encoding another protein. This protein may be another enzyme or a functional domain from another enzyme.

### Host Cells

The present invention also relates to recombinant host cells, comprising a nucleic acid sequence of the invention, which are advantageously used in the recombinant production of the polypeptides. A vector comprising a nucleic acid sequence of the present invention is introduced into a host cell so that the vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be a unicellular microorganism, *e.g*., a prokaryote, or a non-unicellular microorganism, *e.g*., a eukaryote.

Useful unicellular cells are bacterial cells such as gram positive bacteria including, but not limited to, a *Bacillus* cell, or a *Streptomyces* cell, or cells of lactic acid bacteria; or gram negative bacteria such as *E. coli* and *Pseudomonas* sp. Lactic acid bacteria include, but are not limited to, species of the genera *Lactococcus, Lactobacillus, Leuconostoc, Streptococcus, Pediococcus,* and *Enterococcus.*

The introduction of a vector into a bacterial host cell may, for instance, be effected by protoplast transformation (see, *e.g*., Chang and Cohen, 1979, Molecular General Genetics 168: 111-115), using competent cells (see, *e.g*., Young and Spizizin, 1961, Journal of Bacteriology 81: 823-829, or Dubnau and Davidoff-Abelson, 1971, Journal of Molecular Biology 56: 209-221), electroporation (see, *e.g.,* Shigekawa and Dower, 1988, Biotechniques 6: 742-751), or conjugation (see, *e.g*., Koehler and Thorne, 1987, Journal of Bacteriology 169:5771-5278).

The host cell may be a eukaryote, such as a non-human animal cell, an insect cell, a plant cell, or a fungal cell.

In one particular embodiment, the host cell is a fungal cell. "Fungi" as used herein includes the phyla Ascomycota, Basidiomycota, Chytridiomycota, and Zygomycota (as defined by Hawksworth *et al., In, Ainsworth and Bisby's Dictionary of The Fungi,* 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra).*

In another particular embodiment, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in *Biology and Activities of Yeast* (Skinner, F.A., Passmore, S.M., and Davenport, R.R., eds, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell.

The fungal host cell may be a filamentous fungal cell. "Filamentous fungi" include all filamentous forms of the subdivision Eumycota and Oomycota (as defined by Hawksworth *et al.,* 1995, *supra).* The filamentous fungi are characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

Examples of filamentous fungal host cells are cells of species of, but not limited to, *Acremonium, Aspergillus, Fusarium, Humicola, Mucor, Myceliophthora, Neurospora, Penicillium, Thielavia, Tolypocladium,* or *Trichoderma.*

Fungal cells may be transformed by a process involving protoplast formation, transformation of the protoplasts, and regeneration of the cell wall in a manner known *per se.* Suitable procedures for transformation of *Aspergillus* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156 and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

### Methods of Production

The present invention also relates to methods for producing a polypeptide of the present invention comprising (a) cultivating a strain, which in its wild-type form is capable of producing the polypeptide, to produce a supernatant comprising the polypeptide; and (b) recovering the polypeptide. In one embodiment, the strain is of the genus *Thermoascus,* for example of the species *Thermoascus aurantiacus,* such as the strain *Thermoascus aurantiacus* CGMCC No. 0670.

The present invention also relates to methods for producing a polypeptide of the present invention comprising (a) cultivating a host cell under conditions conducive for production of the polypeptide; and (b) recovering the polypeptide.

The present invention also relates to methods for producing a polypeptide of the present invention comprising (a) cultivating a host cell under conditions conducive for production of the polypeptide, wherein the host cell comprises a mutant nucleic acid sequence comprising at least one mutation in nucleotides 25 to 1089, 1 to 1089, 1 to 1344, 25 to 1344, 559 to 1344, or 559 to 1089 of SEQ ID NO:1, in which the mutant nucleic acid sequence encodes a polypeptide which (i) consists of amino acids -178 to 177, -159 to 177, or +1 to 177 of SEQ ID NO:2, or (ii) is a variant of any of the sequences of (i), wherein the variant comprises a substitution, deletion, and/or insertion of one or more amino acids, or (iii) is an allelic variant of any of the sequences of (i), or (iv) is a fragment of any of the sequences of (i).

The present invention also relates to methods for producing a polypeptide of the present invention comprising (a) cultivating a host cell under conditions conducive for production of the polypeptide, wherein the host cell comprises a mutant nucleic acid sequence comprising at least one mutation in nucleotides SEQ ID NO:10, or nucleotides 2-1129; 2-1195; 2-1267; 71-1129; 71-1195; 71-1267; 599-1129; 599-1195; or 599-1267 thereof, or in which the mutant nucleic acid sequence encodes a polypeptide which (i) consists of SEQ ID NO:11, or amino acids -23-353, -23-374, -23-397, 1-353, 1-374, 1-397, 177-353, 177-374, or 177-397 thereof.

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g*., in catalogues of the American Type Culture Collection). If the polypeptide is secreted into the nutrient medium, the polypeptide can be recovered directly from the medium. If the polypeptide is not secreted, it can be recovered from cell lysates.

The polypeptides may be detected using methods known in the art that are specific for the polypeptides. These detection methods may include use of specific antibodies, formation of a protease product, or disappearance of a protease substrate. For example, a protease assay may be used to determine the activity of the polypeptide as described herein.

The resulting polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (*e.g*., ion exchange, affinity, hydrophobic, chromatofocusing, and size exclusion), electrophoretic procedures (*e.g*., preparative isoelectric focusing), differential solubility (*e.g*., ammonium sulfate precipitation), SDS-PAGE, or extraction (see, *e.g.,* Protein Purification, J.-C. Janson and Lars Ryden, editors, VCH Publishers, New York, 1989).

### Plants

The present invention also relates to a transgenic plant, plant part, or plant cell which has been transformed with a nucleic acid sequence encoding a polypeptide having protease activity of the present invention so as to express and produce the polypeptide in recoverable quantities. The polypeptide may be recovered from the plant or plant part. Alternatively, the plant or plant part containing the recombinant polypeptide may be used as such for improving the quality of a food or feed, *e.g*., improving nutritional value, palatability, and rheological properties, or to destroy an antinutritive factor.

In a particular embodiment, the polypeptide is targeted to the endosperm storage vacuoles in seeds. This can be obtained by synthesizing it as a precursor with a suitable signal peptide, see Horvath et al in PNAS, Feb. 15, 2000, vol. 97, no. 4, p. 1914-1919.

The transgenic plant can be dicotyledonous (a dicot) or monocotyledonous (a monocot). Examples of monocot plants are grasses, such as meadow grass (blue grass, Poa), forage grass such as festuca, lolium, temperate grass, such as Agrostis, and cereals, *e.g*., wheat, oats, rye, barley, rice, sorghum, and maize (corn). Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.* Low-phytate plants as described e.g. in US patent no. 5,689,054 and US patent no. 6,111,168 are examples of engineered plants.

Examples of plant parts are stem, callus, leaves, root, fruits, seeds, and tubers. Also specific plant tissues, such as chloroplast, apoplast, mitochondria, vacuole, peroxisomes, and cytoplasm are considered to be a plant part. Furthermore, any plant cell, whatever the tissue origin, is considered to be a plant part.

Also included within the scope of the present invention are the progeny of such plants, plant parts and plant cells.

The transgenic plant or plant cell expressing a polypeptide of the present invention may be constructed in accordance with methods known in the art. Briefly, the plant or plant cell is constructed by incorporating one or more expression constructs encoding a polypeptide of the present invention into the plant host genome and propagating the resulting modified plant or plant cell into a transgenic plant or plant cell.

Conveniently, the expression construct is a nucleic acid construct which comprises a nucleic acid sequence encoding a polypeptide of the present invention operably linked with appropriate regulatory sequences required for expression of the nucleic acid sequence in the plant or plant part of choice. Furthermore, the expression construct may comprise a selectable marker useful for identifying host cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences are determined, for example, on the basis of when, where, and how the polypeptide is desired to be expressed. For instance, the expression of the gene encoding a polypeptide of the present invention may be constitutive or inducible, or may be developmental, stage or tissue specific, and the gene product may be targeted to a specific tissue or plant part such as seeds or leaves. Regulatory sequences are, for example, described by Tague et al., 1988, Plant Physiology 86: 506.

For constitutive expression, the 35S-CaMV promoter may be used (Franck et al., 1980, Cell 21: 285-294). Organ-specific promoters may be, for example, a promoter from storage sink tissues such as seeds, potato tubers, and fruits (Edwards & Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant and Cell Physiology 39: 885-889), a *Vicia faba* promoter from the legumin B4 and the unknown seed protein gene from *Vicia faba* (Conrad et al., 1998, Journal of Plant Physiology 152: 708-711), a promoter from a seed oil body protein (Chen et al., 1998, Plant and Cell Physiology 39: 935-941), the storage protein napA promoter from *Brassica napus,* or any other seed specific promoter known in the art, *e.g*., as described in WO 91/14772. Furthermore, the promoter may be a leaf specific promoter such as the *rbcs* promoter from rice or tomato (Kyozuka et al., 1993, Plant Physiology 102: 991-1000, the chlorella virus adenine methyltransferase gene promoter (Mitra and Higgins, 1994, Plant Molecular Biology 26: 85-93), or the *aldP* gene promoter from rice (Kagaya et al., 1995, Molecular and General Genetics 248: 668-674), or a wound inducible promoter such as the potato pin2 promoter (Xu et al., 1993, Plant Molecular Biology 22: 573-588).

A promoter enhancer element may also be used to achieve higher expression of the protease in the plant. For instance, the promoter enhancer element may be an intron which is placed between the promoter and the nucleotide sequence encoding a polypeptide of the present invention. For instance, Xu *et al.,* 1993, *supra* disclose the use of the first intron of the rice actin 1 gene to enhance expression.

Still further, the codon usage may be optimized for the plant species in question to improve expression (see Horvath et al referred to above).

The selectable marker gene and any other parts of the expression construct may be chosen from those available in the art.

The nucleic acid construct is incorporated into the plant genome according to conventional techniques known in the art, including Agrobacterium-mediated transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

Presently, *Agrobacterium tumefaciens-mediated* gene transfer is the method of choice for generating transgenic dicots (for a review, see Hooykas and Schilperoort, 1992, Plant Molecular Biology 19: 15-38). However it can also be used for transforming monocots, although other transformation methods are generally preferred for these plants. Presently, the method of choice for generating transgenic monocots is particle bombardment (microscopic gold or tungsten particles coated with the transforming DNA) of embryonic calli or developing embryos (Christou, 1992, Plant Journal 2: 275-281; Shimamoto, 1994, Current Opinion Biotechnology 5: 158-162; Vasil et al., 1992, Bio/Technology 10: 667.674). An alternative method for transformation of monocots is based on protoplast transformation as described by Omirulleh et a/., 1993, Plant Molecular Biology 21: 415-428.

Following transformation, the transformants having incorporated therein the expression construct are selected and regenerated into whole plants according to methods well-known in the art.

The present invention also relates to methods for producing a polypeptide of the present invention comprising (a) cultivating a transgenic plant or a plant cell comprising a nucleic acid sequence encoding a polypeptide having protease activity of the present invention under conditions conducive for production of the polypeptide; and (b) recovering the polypeptide.

### Animals

The present invention also relates to a transgenic, non-human animal and products or elements thereof, examples of which are body fluids such as milk and blood, organs, flesh, and animal cells. Techniques for expressing proteins, e.g. in mammalian cells; are known in the art, see e.g. the handbook Protein Expression: A Practical Approach, Higgins and Hames (eds), Oxford University Press (1999), and the three other handbooks in this series relating to Gene Transcription, RNA processing, and Post-translational Recessing. Generally speaking, to prepare a transgenic animal, selected cells of a selected animal are transformed with a nucleic acid sequence encoding a polypeptide having protease activity of the present invention so as to express and produce the polypeptide. The polypeptide may be recovered from the animal, e.g. from the milk of female animals, or the polypeptide may be expressed to the benefit of the animal itself, e.g. to assist the animal's digestion. Examples of animals are mentioned below in the section headed Animal Feed.

To produce a transgenic non-human animal with a view to recovering protease from the milk of the animal, a gene encoding the protease may be inserted into the fertilized eggs of an animal in question, e.g. by use of a transgene expression vector which comprises a suitable milk protein promoter, and the gene encoding protease. The transgene expression vector is is microinjected into fertilized eggs, and preferably permanently integrated into the chromosome. Once the egg begins to grow and divide, the potential embryo is implanted into a surrogate mother, and animals carrying the transgene are identified. The resulting animal can then be multiplied by conventional breeding. The polypeptide may be purified from the animal's milk, see e.g. Meade, H.M. et al (1999): Expression of recombinant proteins in the milk of transgenic animals, Gene expression systems: Using nature for the art of expression. J. M. Fernandez and J. P. Hoeffler (eds.), Academic Press.

In the alternative, in order to produce a transgenic non-human animal that carries in the genome of its somatic and/or germ cells a nucleic acid sequence including a heterologous transgene construct including a transgene encoding the protease, the transgene may be operably linked to a first regulatory sequence for salivary gland specific expression of the protease, as disclosed in WO 2000064247.

### Compositions

In a still further aspect, the present invention relates to compositions comprising a polypeptide of the present invention.

The polypeptide compositions may be prepared in accordance with methods known in the art and may be in the form of a liquid or a dry composition. For instance, the polypeptide composition may be in the form of a granulate or a microgranulate. The polypeptide to be included in the composition may be stabilized in accordance with methods known in the art.

In a particular embodiment, the polypeptide compositions of the invention are protected against acid degradation, e.g. with a view to ensuring a better survival of the polypeptide through the stomach of animals. The protection can be in the form of an acid-stable coating, examples of which can be found in pharmaceutical handbooks.

Examples are given below of preferred uses of the polypeptides or polypeptide compositions of the invention.

### Animal Feed

The present invention is also directed to methods for using the polypeptides having protease activity in animal feed, as well as to feed compositions and feed additives comprising the polypeptides of the invention.

The term animal includes all animals, including human beings. Examples of animals are non-ruminants, and ruminants, such as cows, sheep and horses. In a particular embodiment, the animal is a non-ruminant animal. Non-ruminant animals include monogastric animals, e.g. pigs or swine (including, but not limited to, piglets, growing pigs, and sows); poultry such as turkeys and chicken (including but not limited to broiler chicks, layers); young calves; and fish (including but not limited to salmon).

The term feed or feed composition means any compound, preparation, mixture, or composition suitable for, or intended for intake by an animal.

In the use according to the invention the protease can be fed to the animal before, after, or simultaneously with the diet. The latter is preferred.

In a particular embodiment, the protease, in the form in which it is added to the feed, or when being included in a feed additive, is well-defined. Well-defined means that the protease preparation is at least 50% pure as determined by Size-exclusion chromatography (see Example 12 of WO 01/58275). In other particular embodiments the protease preparation is at least 60, 70, 80, 85, 88, 90, 92, 94, or at least 95% pure as determined by this method.

A well-defined protease preparation is advantageous. For instance, it is much easier to dose correctly to the feed a protease that is essentially free from interfering or contaminating other proteases. The term dose correctly refers in particular to the objective of obtaining consistent and constant results, and the capability of optimising dosage based upon the desired effect.

For the use in animal feed, however, the protease need not be that pure; it may e.g. include other enzymes, in which case it could be termed a protease preparation.

The protease preparation can be (a) added directly to the feed (or used directly in a treatment process of vegetable proteins), or (b) it can be used in the production of one or more intermediate compositions such as feed additives or premixes that is subsequently added to the feed (or used in a treatment process). The degree of purity described above refers to the purity of the original protease preparation, whether used according to (a) or (b) above.

Protease preparations with purities of this order of magnitude are in particular obtainable using recombinant methods of production, whereas they are not so easily obtained and also subject to a much higher batch-to-batch variation when the protease is produced by traditional fermentation methods.

Such protease preparation may of course be mixed with other enzymes.

In a further particular embodiment, the protease for use according to the invention is capable of:
Increasing digested protein, degree of hydrolysis (DH) and/or solubilised protein according to the full *in vitro* monogastric digestion model of Example 6 herein;
increasing digested protein, DH and/or solubilised protein according to the intestinal part of the *in vitro* monogastric digestion model of Example 6 herein; and/or
increasing protein solubility, protein digestibility and/or DH, according to the aquaculture *in vitro* digestion model of Example 7 herein.

The term vegetable proteins as used herein refers to any compound, composition, preparation or mixture that includes at least one protein derived from or originating from a vegetable, including modified proteins and protein-derivatives. In particular embodiments, the protein content of the vegetable proteins is at least 10, 20, 30, 40, 50, or 60% (w/w).

Vegetable proteins may be derived from vegetable protein sources, such as legumes and cereals, for example materials from plants of the families *Fabaceae (Leguminosae), Cruciferaceae, Chenopodiaceae,* and *Poaceae,* such as soy bean meal, lupin meal and rapeseed meal.

In a particular embodiment, the vegetable protein source is material from one or more plants of the family *Fabaceae,* e.g. soybean, lupine, pea, or bean.

In another particular embodiment, the vegetable protein source is material from one or more plants of the family *Chenopodiaceae,* e.g. beet, sugar beet, spinach or quinoa.

Other examples of vegetable protein sources are rapeseed, and cabbage.

Soybean is a preferred vegetable protein source.

Other examples of vegetable protein sources are cereals such as barley, wheat, rye, oat, maize (corn), rice, and sorghum.

The treatment according to the invention of vegetable proteins with at least one protease of the invention results in an increased solubilisation of vegetable proteins.

The following are examples of % solubilised protein obtainable using the proteases of the invention: At least 100.0%, or 100.1, 100.2, 100.2, 100.4, 100.5, 100.6, 100.7, 100.8, 100.9, 101.0, 101.1, 101.2, 101.3, or at least 101.4%, relative to a blank, and reference being had to the full *in vitro* monogastric digestion model of Example 6 herein.

The following are examples of % digestible protein obtainable using the proteases of the invention: At least 100%, or 101, 102, 103, or at least 104%, relative to a blank, and reference being had to the full *in vitro* monogastric digestion model of Example 6 herein.

The following are examples of i) % solubilised; and ii) % digested protein obtainable using the proteases of the invention: At least 100%, or 101, 102, or at least 103%, relative to a blank, and reference being had to the intestinal part of the *in vitro* monogastric digestion model of Example 6 herein.

The following are examples of % degree of hydrolysis (DH) obtainable using the proteases of the invention: At least 100%, or 101, 102, 103, 104, 105, 106, 107, 108, 109, 110, 111, or at least 112%, relative to a blank, and reference being had to the aquaculture *in vitro* digestion model of Example 7 herein.

The following are examples of i) % solubilised; and ii) % digested protein obtainable using the proteases of the invention: At least 100%, or 101, 102, 103, 104, 105, 106, 107, 108, 109, 110, 111, 112, or at least 113%, relative to a blank, and reference being had to the aquaculture *in vitro* digestion model of Example 7 herein.

The term solubilisation of proteins basically means bringing protein(s) into solution. Such solubilisation may be due to protease-mediated release of protein from other components of the usually complex natural compositions such as feed. Solubilisation can be measured as an increase in the amount of solubilised proteins, by reference to blank samples treated with pepsin and pancreatin only (see Examples 6 and 7 herein).

The term digestion of proteins basically means proteolytic hydrolysis of solubilised proteins by action of proteases so that low-molecular mass peptides and amino acids are formed. Digestion of solubilised proteins can be measured as an increased amount of solubilised peptides and amino acids with a molecular mass equal to or smaller than 1500 dalton, by reference to blank samples treated with pepsin and pancreatin only (see Examples 6 and 7 herein).

In a particular embodiment of a treatment process the protease(s) in question is affecting (or acting on, or exerting its solubilising influence on) the vegetable proteins or protein sources. To achieve this, the vegetable protein or protein source is typically suspended in a solvent, e.g. an aqueous solvent such as water, and the pH and temperature values are adjusted paying due regard to the characteristics of the enzyme in question. For example, the treatment may take place at a pH-value at which the activity of the actual protease is at least 50, or 60, or 70, or 80 or 90%. Likewise, for example, the treatment may take place at a temperature at which the activity of the actual protease is at least 50, or 60, or 70, or 80 or 90%. The above percentage activity indications are relative to the maximum activities. The enzymatic reaction is continued until the desired result is achieved, following which it may or may not be stopped by inactivating the enzyme, e.g. by a heat-treatment step.

In another particular embodiment of a treatment process of the invention, the protease action is sustained, meaning e.g. that the protease is added to the vegetable proteins or protein sources, but its solubilising influence is so to speak not switched on until later when desired, once suitable solubilising conditions are established, or once any enzyme inhibitors are inactivated, or whatever other means could have been applied to postpone the action of the enzyme.

In one embodiment the treatment is a pre-treatment of animal feed or vegetable proteins for use in animal feed, i.e. the proteins are solubilised before intake.

The term improving the nutritional value of an animal feed means improving the availability of the proteins, thereby leading to increased protein extraction, higher protein yields, and/or improved protein utilisation. The nutritional value of the feed is therefore increased, and the growth rate and/or weight gain and/or feed conversion (i.e. the weight of ingested feed relative to weight gain) of the animal is/are improved.

The protease can be added to the feed in any form, be it as a relatively pure protease, or in a mixture with other components intended for addition to animal feed, i.e. in the form of animal feed additives, such as the so-called pre-mixes for animal feed.

In a further aspect the present invention relates to compositions for use in animal feed, such as animal feed, and animal feed additives, e.g. premixes.

Apart from the protease of the invention, the animal feed additives of the invention contain at least one fat-soluble vitamin, and/or at least one water soluble vitamin, and/or at least one trace mineral, and/or at least one macro mineral.

Further, optional, feed-additive ingredients are colouring agents, aroma compounds, stabilisers, antimicrobial peptides, and/or at least one other enzyme selected from amongst phytases EC 3.1.3.8 or 3.1.3.26; xylanases EC 3.2.1.8; galactanases EC 3.2.1.89; and/or beta-glucanases EC 3.2.1.4.

In a particular embodiment these other enzymes are well-defined (as defined above for protease preparations).

Examples of antimicrobial peptides (AMP's) are CAP18, Leucocin A, Tritrpticin, Protegrin-1, Thanatin, Defensin, and Ovispirin such as Novispirin (Robert Lehrer, 2000), Plectasins, and Statins, including the compounds and polypeptides disclosed in PCT/DK02/00781 and PCT/DK02/00812, as well as variants or fragments of the above that retain antimicrobial activity.

Examples of antifungal polypeptides (AFP's) are the *Aspergillus giganteus,* and *Aspergillus niger* peptides, as well as variants and fragments thereof which retain antifungal activity, as disclosed in WO 94/01459 and WO 02/090384.

Usally fat- and water-soluble vitamins, as well as trace minerals form part of a so-called premix intended for addition to the feed, whereas macro minerals are usually separately added to the feed. Either of these composition types, when enriched with a protease of the invention, is an animal feed additive of the invention.

In a particular embodiment, the animal feed additive of the invention is intended for being included (or prescribed as having to be included) in animal diets or feed at levels of 0.01 to 10.0%; more particularly 0.05 to 5.0%; or 0.2 to 1.0% (% meaning g additive per 100 g feed). This is so in particular for premixes.

The following are non-exclusive lists of examples of these components:
Examples of fat-soluble vitamins are vitamin A, vitamin D3, vitamin E, and vitamin K, e.g. vitamin K3.

Examples of water-soluble vitamins are vitamin B12, biotin and choline, vitamin B1, vitamin B2, vitamin B6, niacin, folic acid and panthothenate, e.g. Ca-D-panthothenate.

Examples of trace minerals are manganese, zinc, iron, copper, iodine, selenium, and cobalt.

Examples of macro minerals are calcium, phosphorus and sodium.

The nutritional requirements of these components (exemplified with poultry and piglets/pigs) are listed in Table A of WO 01/58275. Nutritional requirement means that these components should be provided in the diet in the concentrations indicated.

In the alternative, the animal feed additive of the invention comprises at least one of the individual components specified in Table A of WO 01/58275. At least one means either of, one or more of, one, or two, or three, or four and so forth up to all thirteen, or up to all fifteen individual components. More specifically, this at least one individual component is included in the additive of the invention in such an amount as to provide an in-feed-concentration within the range indicated in column four, or column five, or column six of Table A.

The present invention also relates to animal feed compositions. Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore such fish diets usually have a crude fat content of 200-310 g/kg.

An animal feed composition according to the invention has a crude protein content of 50-800 g/kg, and furthermore comprises at least one protease as claimed herein.

Furthermore, or in the alternative (to the crude protein content indicated above), the animal feed composition of the invention has a content of metabolisable energy of 10-30 MJ/kg; and/or a content of calcium of 0.1-200 g/kg; and/or a content of available phosphorus of 0.1-200 g/kg; and/or a content of methionine of 0.1-100 g/kg; and/or a content of methionine plus cysteine of 0.1-150 g/kg; and/or a content of lysine of 0.5-50 g/kg.

In particular embodiments, the content of metabolisable energy, crude protein, calcium, phosphorus, methionine, methionine plus cysteine, and/or lysine is within any one of ranges 2, 3, 4 or 5 in Table B of WO 01/58275 (R. 2-5).

Crude protein is calculated as nitrogen (N) multiplied by a factor 6.25, i.e. Crude protein (g/kg)= N (g/kg) × 6.25. The nitrogen content is determined by the Kjeldahl method (A.O.A.C., 1984, Official Methods of Analysis 14th ed., Association of Official Analytical Chemists, Washington DC).

Metabolisable energy can be calculated on the basis of the NRC publication Nutrient requirements in swine, ninth revised edition 1988, subcommittee on swine nutrition, committee on animal nutrition, board of agriculture, national research council. National Academy Press, Washington, D.C., pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.

The dietary content of calcium, available phosphorus and amino acids in complete animal diets is calculated on the basis of feed tables such as Veevoedertabel 1997, gegevens over chemische samenstelling, verteerbaarheid en voederwaarde van voedermiddelen, Central Veevoederbureau, Runderweg 6, 8219 pk Lelystad. ISBN 90-72839-13-7.

In a particular embodiment, the animal feed composition of the invention contains at least one vegetable protein or protein source as defined above.

In still further particular embodiments, the animal feed composition of the invention contains 0-80% maize; and/or 0-80% sorghum; and/or 0-70% wheat; and/or 0-70% Barley; and/or 0-30% oats; and/or 0-40% soybean meal; and/or 0-10% fish meal; and/or 0-20% whey.

Animal diets can e.g. be manufactured as mash feed (non pelleted) or pelleted feed. Typically, the milled feed-stuffs are mixed and sufficient amounts of essential vitamins and minerals are added according to the specifications for the species in question. Enzymes can be added as solid or liquid enzyme formulations. For example, a solid enzyme formulation is typically added before or during the mixing step; and a liquid enzyme preparation is typically added after the pelleting step. The enzyme may also be incorporated in a feed additive or premix.

The final enzyme concentration in the diet is within the range of 0.01-200 mg enzyme protein per kg diet, for example in the range of 5-30 mg enzyme protein per kg animal diet. In particular embodiments, the enzyme concentration in the diet is 0.1-180; 0.1-160; 0.1-150; 0.2-125; 0.2-100; 1-180; 2-160; 5-150; 10-100; 10-90; 10-80; 10-70; or 10-50 mg enzyme protein per kg animal diet.

In particular embodiments of the method of the invention for treating vegetable proteins, a further step of adding phytase is also included. And in further particular embodiments, in addition to the combined treatment with phytase and protease, further enzymes may also be added, wherein these enzymes are selected from the group comprising other proteases, phytases, lipolytic enzymes, and glucosidase/carbohydrase enzymes. Examples of such enzymes are indicated in WO95/28850.

The protease should of course be applied in an effective amount, i.e. in an amount adequate for improving solubilisation and/or improving nutritional value of feed. It is at present contemplated that the enzyme is administered in one or more of the following amounts (dosage ranges): 0.01-200; or 0.01-100; or 0.05-100; or 0.05-50; or 0.10-10 - all these ranges being in mg protease protein per kg feed (ppm).

For determining mg protease protein per kg feed, the protease is purified from the feed composition, and the specific activity of the purified protease is determined using a relevant assay (see under protease activity, substrates, and assays). The protease activity of the feed composition as such is also determined using the same assay, and on the basis of these two determinations, the dosage in mg protease protein per kg feed is calculated.

The same principles apply for determining mg protease protein in feed additives. Of course, if a sample is available of the protease used for preparing the feed additive or the feed, the specific activity is determined from this sample (no need to purify the protease from the feed composition or the additive).

The present invention is further described by the following examples which should not be construed as limiting the scope of the invention.

### Examples

### Reagents, Media, and Equipment

### Reagents:

Unless otherwise specified, the chemicals used were commercial products of at least reagent grade.

AZCL-casein (Megazyme Cat. No. I-AZCAS). Azurine-Cross-Linked casein is prepared by dyeing and crosslinking highly purified casein and used for assay of endo-protease. It is supplied as a fine powder which is insoluble in buffered solution, but it rapidly hydrates to form gel particles which are readily and rapidly hydrolysed by endo-proteases, thus releasing the soluble dye-labeled fragment.
pNA-substrates:
(AAPF) Ala-Ala-Pro-Phe-pNA (Sigma S-7388)
(GGF) Gly-Gly-Phe-pNA (Sigma S-1899)
(FGL) Phe-Gly-Leu-pNA (Sigma S-6768)
BA-pNA (Sigma B-4875)
(AAPE) Ala-Ala-Pro-Glu-pNA (BACHEM C-1710)

SSI (Streptomyces Subtilisin Inhibitor): SSI was purified from a *Streptomyces albogriseolus* FERM P-1205 (S-3253) fermentation supernatant by chromatography. The SSI preparation had a purity above 95% as determined by SDS-PAGE. Alternatively, SSI can be obtained from Wako in Japan, catalog no. 303-05201, manufactured by Daiwa Kasei K.K. (see eg: http://search.wako-chem.co.jp/lifedb_e/)ifedocs_e/44834.asp).
EDTA (Gibco BRL Cat.No. 15576-028)
IPTG (Promega, Cat. No. V3951)
X-gal (Promega, Cat. No. V3941)
Ampicillin Sodium Salt (GIBCOL., Cat.No. 11593-019)
LMP agarose (Promega, Cat. No. V2111)

Trypsin designates a trypsin-like protease derived from a strain of *Fusarium oxysporum.* Its preparation is described in EP 471265, Examples 1 and 2 ("protease II").

SAVINASE^{™} is a subtilisin protease derived from *Bacillus clausii* (previously *Bacillus lentus* NCIB 10309), commercially available from Novozymes A/S, Krogshoejvej, DK-2880 Bagsvaerd, Denmark. Its preparation is described in US patent No. 3723250.
Pepsin (Sigma P-7000; 539 U/mg, solid)
Pancreatin (Sigma P-7545; 8xU.S.P. (US Pharmacopeia))
Cytochrome C (12500 dalton, Boehringer Mannheim 103870)
Aprotinin (6500 dalton, Sigma A-1153)
Gastrin I (2126 dalton, Sigma G-1276)
Substance P (1348 dalton, Sigma S-6883)

### Media:

Buffer system (pH 3 to pH 11): 100mM succinic acid, 100mM HEPES, 100mM CHES, 100mM CABS, 1 mM CaCl₂, 150mM KCl, 0.01% Triton^{®} X-100 adjusted to pH-values 2.0, 2.5, 3.0, 3.5, 4.0, 4.5, 5.0, 6.0, 7.0, 8.0, 9.0, 10.0 or 11.0 with HCl or NaOH (herein for short designated "the succinic acid buffer system").

Borax/NaH₂PO₄, pH9: Prepared by mixing 8.25ml 0.05M Borax with 1.75ml 0.1M NaH₂PO₄.
1×TAE buffer: 0.04M Tris-acetate; 0.001 M EDTA.
0.5×TBE buffer: 0.045M Tris-borate; 0.001 M EDTA.
1 mM pNA substrates: Weigh 5mg pNA substrate and dissolve into 100µl DMSO, then dilute with 10ml Borax/NaH₂PO₄ pH9.0 buffer

### CBH1 medium:

| | | | |
|---|---|---|---|
| Avicel | 25 g | (NH₄)₂SO₄ | 1.4 g |
| KH₂PO₄ | 2 g | Urea | 0.3g |
| CaCl₂.2H₂O | 0.3g | MgSO₄.7H₂O | 0.3g |
| FeSO₄.7H₂O | 5mg | MnSO₄.H₂O | 1.6mg |
| Peptone | 1g | Yeast Extract | 10g |
| TWEEN80 | 1ml | Glucose | 5g |
| H₂O | 1000 ml | | |

80 ml in 500 ml Erlenmeyer flask, autoclave 20 minutes under 121°C.

LB liquid medium: To 950ml of deionized H₂O, add: 10g bacto-tryptone, 5g bacto-yeast extract, 10g NaCl. Shake until the solutes have dissolved. Adjust the pH to 7.0 with 5N NaOH (~0.2ml). Adjust the volume of the solution to 1 liter with deionized H₂O. Sterilize by autoclaving for 20 minutes at 15lb/sq. in. on liquid cycle.

LB plates with ampicillin/IPTG/X-Gal: Add 15g agar to 1 liter of LB medium. Add ampicillin to a final concentration of 100µg/ml, then supplement with 0.5mM IPTG and 80µg/ml X-gal and pour the plates.

1% LMP agarose gel: Add 1g LMP agarose into 100ml 1 × TAE buffer.

IPTG stock solution (0.1M):
Add distilled water to 1.2g IPTG to 50ml final volume, filter-sterilize and store at 4°C.

Maize/-SBM diet (SBM = Soybean meal): The maize/-SBM ratio is 6:4 (w/w), the protein content 43% (w/w) in SBM and 8.2% (w/w) in maize meal. The total amount of protein in 10g maize-SBM diet is 2.21g.

Extruded SBM: The protein content is 45%. The total amount of protein in 10g extruded SBM is thus 4.5 g.

### Equipment, including various Kits:

5K membrane (Millipore BIOMAX-5, 13442AM)
0.45µm filter (Nalgene 190-2545)
Q Sepharose FF column (Amersham Pharmacia 17-0510-01)
Superdex75 column (Amersham Pharmacia 17-1047-01)
IEF-gel (Amersham Pharmacia 80-1124-80)
Thermomixer comfort (Eppendorf)
RNeasy Mini Kit (QIAGEN, Cat.No.74904)
3' RACE Kit (GIBCO, Cat.No.18373-019) including Adapter primer, and AUAP
dNTP mix (100mM, Promega, Cat. No. U1330)
TaqDNA polymerase system (Promega, Cat. No. M1661) including PCR buffer (200mM Tris-HCl (pH8.4), 500mM KCI)
PCR Preps DNA Purification System (Promega, Cat.No.A7170)
pGEM-T Vector System (Promega, Cat.No.A3600) including T4 DNA Ligase 2XBuffer
JM109 high efficiency competent cells (Promega, Cat. No.L1001)
Minipreps DNA Purification System (Promega, Cat.No.A7100)
BigDye Terminator Cycle Sequencing Ready Reaction Kit (PE Applied Biosystems, Cat. No. 4303149)
ABI Prism 377 DNA sequencer (PE)
5'RACE system (GIBCO, CAT.NO.18374-058) including Abridged Anchor Primer
pfu DNA polymerase system (Promega, Cat. No. M7741)
0.45 µm polycarbonate filters (Sartorius)
Superdex Peptide PE (7.5 × 300 mm) gelfiltration column (Global)
Spectrophotometer DU7500 (Beckman)
GeneAmp PCR System 9700(PE)
Vac-Man Jr. Laboratory Vacuum Manifold (Promega, Cat. No. A7660)

### Example 1: Protease assays

The following assays for protease activity were used:

### AZCL-casein assay

A solution of 0.2% of the blue substrate AZCL-casein is suspended in Borax/NaH₂PO₄ buffer pH9 while stirring. (For the pH profile part of Example 4, the buffer system pH3 to pH11 was used instead). The solution is distributed while stirring to microtiter plate (100µl to each well), 30µl enzyme sample is added and the plates are incubated in an Eppendorf Thermomixer for 30 minutes at 45°C and 600rpm. Denatured enzyme sample (100°C boiling for 20min) is used as a blank. After incubation the reaction is stopped by transferring the microtiter plate onto ice and the coloured solution is separated from the solid by centrifugation at 3000rpm for 5 minutes at 4°C. 60µl of supernatant is transferred to a microtiter plate and the absorbance at 595nm is measured using a BioRad Microplate Reader.

### pNA-assay

50µl protease-containing sample is added to a microtiter plate and the assay is started by adding 100µl 1mM pNA substrate (5 mg dissolved in 100 µl DMSO and further diluted to 10 ml with Borax/NaH₂PO₄ buffer pH9.0). The increase in OD₄₀₅ at room temperature is monitored as a measure of the protease activity.

### Example 2: Cultivation of Thermoascus aurantiacus CGMCC No. 0670

*Thermoascus aurantiacus* CGMCC No. 0670 was grown at 45°C for 60 hours in shake flasks with the CBH1 medium. The culture broth was harvested by centrifugation (7000 rpm for 20 minutes at 4°C). A total of 1500 ml culture broth was obtained.

### Example 3: Purification of the protease of Thermoascus aurantiacus CGMCC No. 0670

1500ml supernatant from Example 2 was precipitated with ammonium sulfate (80% saturation) and re-dissolved in 40 ml 25 mM Tris-HCl, pH7.4 buffer. The resulting solution was ultrafiltrated with a 5K membrane to remove salts and change buffer to 25 mM Tris-HCl, pH7.4, following which it was filtered through a 0.45µm filter. The final volume was 30 ml. The solution was applied to a 20 ml Q Sepharose FF column equilibrated in 25 mM Tris-HCl, pH7.4, and the proteins were eluted with a linear NaCl gradient (0 - 0.4M). Fractions from the column were analyzed for protease activity on AZCL-casein at pH 9.0, with or without SSI. Fractions with protease activity not inhibited by SSI were pooled. Then the pooled solution was applied to a Superdex75 column equilibrated with 25 mM Tris-HCl, pH7.4, and the proteins were eluted with the same buffer. Protease-containing fractions were analyzed by SDS-PAGE and pure fractions were pooled.

The purity of the purified protease was checked by SDS-page and on an IEF gel. The sample contained only one protease which was designated AP025. The molecular weight is around 23 kDa and the pl is pH8.5.

### Example 4: Characterization of the protease of Thermoascus aurantiacus CGMCC No. 0670

### Inhibition test

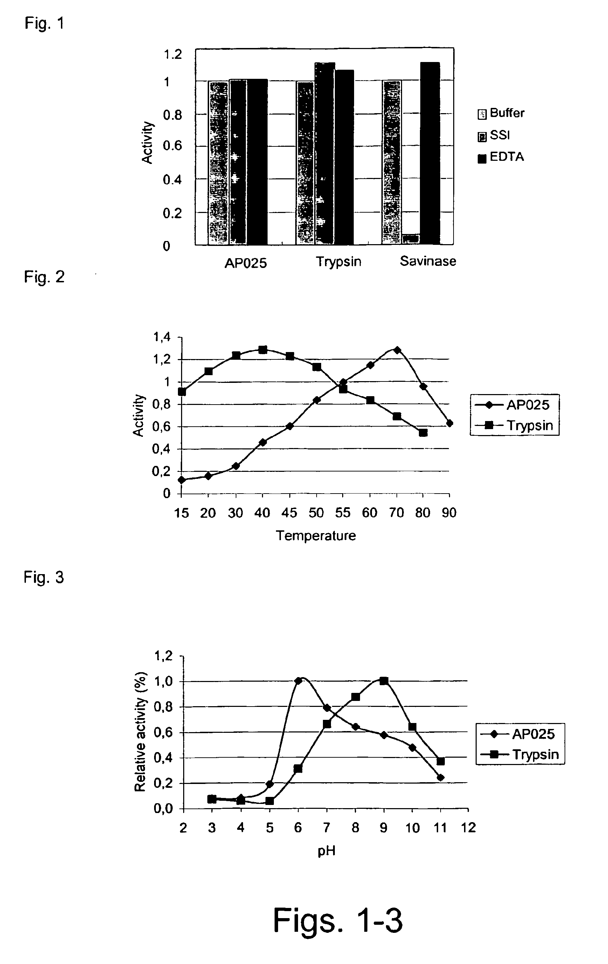

Purified protease AP025 was tested for inhibition by various inhibitors, including SSI and EDTA. The protease was incubated with 1.12 mg/ml SSI, or 0.5M EDTA for 5 minutes at room temperature, and residual activity was determined using the AZCL-casein assay of Example 1 (pH9, 45°C). Trypsin and SAVINASE™ were included as controls. As is apparent from the results shown in Fig. 1, the activity of AP025 seems to be neither inhibited by SSI nor by EDTA.

### Temperature profile

The relationship between temperature and protease activity was evaluated using AZCL-casein assay (20 minutes with shaking in thermomixer). The resulting temperature profile is shown in Fig. 2.

The protease AP025 is active in a wide range of temperatures from 20-90°C and appears to have its optimum temperature around 70°C. But even at 90°C. the activity is at least 60%, relative to the activity at 55°C.

### pH profile

The relationship between pH and protease activity was evaluated using the AZCL-casein assay of Example 1 (and the buffer system pH 3 to pH 11). The resulting pH profile is shown in Fig. 3.

The protease AP025 appears to have activity in a broad pH-range from pH 5-10. The optimum pH is around 6.

### pH stability

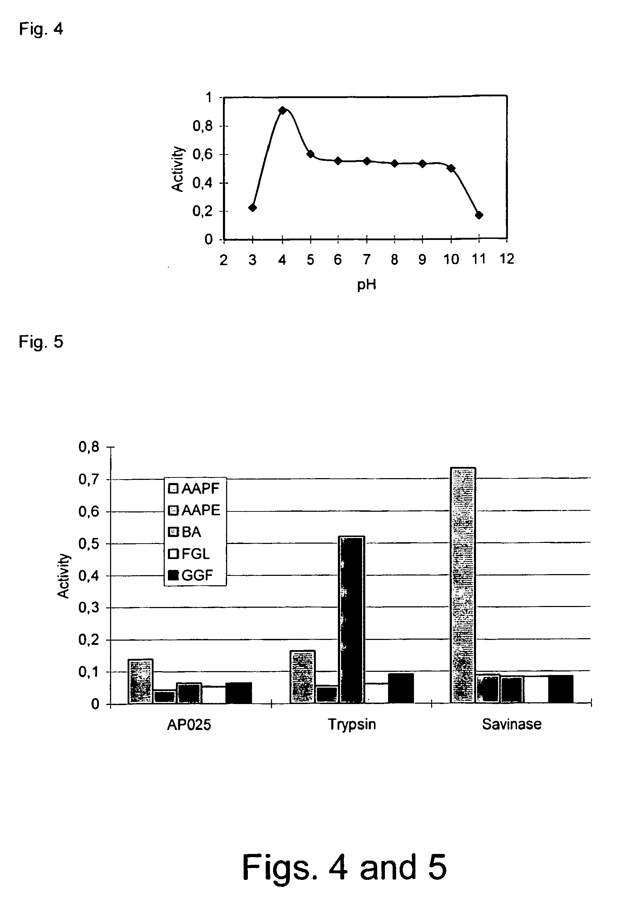

For pH stability measurements, a mixture of 15 ml enzyme sample and 200 ml buffer pH 3,4,5,6,7,8,9,10,11 was incubated at 37°C for 2 hours, and then the residual enzyme activity was assayed using the AZCL-casein of Example 1 (add 900ul AZCL-casein in Borax/NaH₂PO₄ buffer, pH9.0, incubate the mixture at 45°C, 1200rpm for 10 min; then OD was measured at 595 nm). The results are shown in Fig. 4. The protease appears to be stable in a very wide pH-range of pH 4-10, but it is optimally stable around pH 4.

### pNA substrate specificity

The substrate specificity of AP025 was evaluated using a few pNA substrates including AAPF, AAPE, GGF, BA and FGL, with SAVINASE™ and Trypsin as references.

From the results shown in Fig. 5, it appears that AP025 has no substantial activity on any of these substrates.

### N-terminal sequencing

The N-terminal amino acid sequence of the protease AP025 was: QRISSCSGSRQSALTTALRN (SEQ ID NO:3).

### Example 5: Cloning of the gene encoding the protease of Thermoascus aurantiacus CGMCC 0670

### Fungal strain and its growth

*Thermoascus aurantiacus* CGMCC No. 0670 was grown at 45°C, 165 rpm for 3 days in CBH1 medium. The mycelium was harvested by centrifugation at 7000 rpm for 30 minutes. The harvested mycelium was stored at -80°C before use for RNA extraction.

### Extraction of total RNA

Total RNA was extracted from 100 mg mycelium using the RNeasy Mini Kit.

### Degenerate primers

The following degenerate primers were designed based on part of the N-terminal amino acid sequence, QSALTTA:
T025-3 5' CA(A/G) TC(T/C/A/G) GC(T/C/A/G) CT(T/C/A/G) AC(T/C) AC(A/G) GC 3' (SEQ ID NO: 4)
T025-12 5' CA(A/G) AG(T/C) GC(T/C/A/G) CT(T/C/A/G) AC(A/G) AC(A/G) GC 3' (SEQ ID NO: 5)

### Cloning of the 3'-end of AP025

The 3' RACE Kit was used to synthesize the cDNA of AP025. About 5mg total RNA was used as a template and the Adapter Primer was used to synthesize the first strand of cDNA. Then the cDNA of AP025 was PCR-amplified using the above degenerate primers.

The PCR reaction system and conditions were as follows:

| | |
|---|---|
| 10 x PCR buffer | 5µl |
| 25mM MgCl₂ | 3µl |
| 10mM dNTP mix | 1µl |
| 3'Primer (T025-3, T025-12; 10µM) | 1 µl |
| AUAP(10µM) | 1µl |
| TaqDNA polymerase | 0.5µl |
| cDNA synthesis reaction | 2µl |
| Add autoclaved, | |
| distilled water to | 50µl |

### Conditions

| | | |
|---|---|---|
| 94°C | 3 min | |
| 94°C | 40sec | |
| 55°C | 40sec | 30 cycles |
| 72°C | 1 min | |
| 72°C | 10min | |

Gel analysis of the PCR product revealed a specific band corresponding to a fragment of about 800bp, using primers AP025-3 and AP025-12. The products were recovered from 1% LMP agarose gel, purified by incubation in a 70°C bath, followed by using the PCR Preps DNA Purification System. The concentrations of purified products were determined by measuring the absorbance of A₂₆₀ and A₂₈₀ in a spectrophotometer. Then these purified fragments were ligated to the pGEM-T Vector using the corresponding Promega Kit:

| | |
|---|---|
| T4 DNA Ligase 2XBuffer | 1µl |
| pGEM-T Vector (50ng) | 1µl |
| PCR product | 40ng |
| T4 DNA Ligase (3 Weiss units/µl) | 1µl |
| dH2O to a final volume of | 10µl |

The reactions were incubated overnight at 4°C.

2-4µl of the ligation products were transformed into 50µl JM109 high efficiency competent cells by the "heat shock" method (J. Sambrook, E.F.Fritsch, T.Maniatis (1989) Molecular Cloning 1.74, 1.84).

Transformation cultures were plated onto LB plates with ampicillin/IPTG/X-Gal, and these plates were incubated overnight at 37°C. Recombinant clones were identified by colour screening on indicator plates and colony PCR screening as follows:

### Colony PCR system:

| | |
|---|---|
| 10 x PCR buffer | 5µl |
| 25mM MgCl₂ | 3µl |
| 10mM dNTP mix | 1µl |
| 3'Primer (10µM, T025-3 or T025-12) | 2µl |
| AUAP (10µM) | 2µl |
| TaqDNA polymerase | 0.5µl |
| Add autoclaved, distilled water to | 50µl |

Dip a white colony with a tip and pipet the colony into PCR mixture as the template.

### PCR Conditions

| | | |
|---|---|---|
| 94°C | 3 min | |
| 94°C | 40sec | |
| 55°C | 40sec | 30 cycles |
| 72°C | 1.5 min | |
| 72°C | 10min | |

The positive clones were inoculated into 3ml LB liquid medium and incubated overnight at 37°C with shaking (about 250rpm). The cell pellet was separated by centrifugation for 5min at 10,000 x g, and plasmid samples were prepared from the cell pellet by using Minipreps DNA Purification System. Finally, the plasmids were sequenced using the BigDye Terminator Cycle Sequencing Ready Reaction Kit and the AB1377 sequencer. The sequencing reaction was as follows:

| | |
|---|---|
| Terminator Ready Reaction Mix | 8 µl |
| Plasmid DNA | 1.0-1.5µg |
| Primer | 3.2pmol |
| dH2O to a final volume of | 10 µl |

The sequencing results showed that the PCR band obtained by using primer AP025-3, as well as primer AP025-12, is the 3'end sequence of AP025.

### Cloning of the 5'-end of AP025

Based on the 3'-end sequence of AP025, we designed three specific primers that were used for cloning the 5'-end of the sequence.

| | | |
|---|---|---|
| AP025-5'-1 5' | AAG GTA TAT GGC ATT CGC AT | 3' (SEQ ID NO:6) |
| AP025-5'-2 5' | GCA GCC TGG TAG CCA TAC | 3' (SEQ ID NO:7) |
| AP025-5'-3 5' | TTG ATC CTG AGC GTG ACA G | 3' (SEQ ID NO:8) |

The 5'RACE system was used to synthesize the 5'-end fragment of AP025. 5 µg total RNA and primer 5'-1 was added for synthesis of the first strand. Then the cDNA was purified with DNA Purification System (provided by the 5'RACE system) and a polyC tail was added to the 3'-end of the cDNA. The primer 5'-2 was used for the second strand synthesis.

The following system and conditions of PCR of dC-tailed cDNA were used:

| | |
|---|---|
| 10 x PCR buffer (200mM Tris-HCl (pH8.4), 500mM KCI) | 5µl |
| 25mM MgCl₂ | 3µl |
| 10mM dNTP mix | 1µl |
| 5'Primer (10µM) (AP025-5'-1/2/3) | 2µl |
| Abridged Anchor Primer | 2µl |
| TaqDNA polymerase | 0.5µl |
| dC-tailed cDNA | 5µl |
| Add autoclaved, distilled water to | 50µl |

### PCR Conditions

| | | |
|---|---|---|
| 94°C | 2min | |
| 94°C | 40sec | |
| 55°C | 40sec | 30 cycles |
| 72°C | 1 min | |
| 72°C | 10min | |

To get a specific product we amplified the 5' end using AP025-5'-3 primer by nested PCR (Frohman, M.A. (1990) PCR Protocols: A Guide to Methods and Applications (Innis, M.A., Gelfand, D.H., Sninsky, J.J., and White, T.J., eds.) p. 28, Academic Press, San Diego.

| | |
|---|---|
| Sterilized, distilled water | 33.5µl |
| 10 X PCR buffer | 5.0µl |
| 25 mM MgCl₂ | 3.0µl |
| 10 mM dNTP mix | 1.0µl |
| primer AP025 5' -3 (10µM) | 1.0µl |
| AUAP (10mM, provided by 5'RACE system) | 1.0µl |
| dilution of primary PCR product (1:100) | 5.0µl |
| Taq DNA polymerase (2 to 5 units/µl) | 0.5µl |

### PCR Conditions

| | | |
|---|---|---|
| 94°C | 2min | |
| 94°C | 40sec | |
| 58°C | 40sec | 30 cycles |
| 72°C | 1 min | |
| 72°C | 10min | |

Using primer AP025-5'-3 in the 5'RACE system, a specific band was obtained with the size of a 1000bp fragment. The PCR-products were purified, ligated into pGEM-T-vector, and transformed to JM109 competent cells, and sequenced. Sequencing confirmed that the 5'end fragment of AP025 had been cloned.

### Cloning of the complete AP025 gene:

A primer for full length cloning was designed on the basis of the above 3'- and 5'-end sequences:
AP025-CDS-2 5' AAG TCT ACC CAG TAT CCT GT 3' (SEQ ID NO:9)

Primer AP025-CDS-2 and AUAP was used for amplifying the full length gene from cDNA of AP025. The following PCR reaction system and conditions were used:

| | |
|---|---|
| 10 x PCR buffer | 5µl |
| 25mM MgCl₂ | 3µl |
| 10mM dNTP mix | 11µl |
| Primer AP025-CDS-2 (10µM) | 1µl |
| AUAP(10µM) | 1µl |
| pfu DNA polymerase | 0.5µl |
| cDNA synthesis reaction | 2µl |
| Add autoclaved, distilled water to | 50µl |

### Conditions:

| | | |
|---|---|---|
| 95°C | 2min | |
| 95°C | 40sec | |
| 59°C | 30sec | 30 cycles |
| 72°C | 3.5min | |
| 72°C | 5min | |

A specific band with the size of about 1.4kb was the result of this amplification. A polyA tail was added using Taq DNA polymerase and incubation at 72°C for 30min. The dA-tailed fragment was recovered from the gel with PCR Preps DNA Purification System. Then the purified fragment was ligated into the pGEM-T Vector, and transformed it into the competent cells (JM109). Some positive clones were screened by colony PCR.

### Colony PCR system:

| | |
|---|---|
| 10 x PCR buffer | 5µl |
| 25mM MgCl₂ | 3µl |
| 10mM dNTP mix | 1µl |
| AP025-CDS-2(10µM. AP025-3 or AP025-12) | 2µl |
| AUAP(10µM) | 2µl |
| TaqDNA polymerase | 0.5µl |
| Add autoclaved, distilled water to | 50µl |

Dip a white colony with a tip and pipet the colony into PCR mixture as the template.

### Conditions

| | | |
|---|---|---|
| 94°C | 3 min | |
| 94°C | 40sec | |
| 55°C | 40sec | 30 cycles |
| 72°C | 1.5 min | |
| 72°C | 10min | |

Then the plasmid was extracted from these clones with Minipreps DNA Purification System. The plasmid was sequenced using the BigDye Terminator Cycle Sequencing Ready Reaction Kit, and the full-length sequence was obtained.

Sequence analysis of the cDNA clone showed that the sequence contains a coding region of 1065 nucleotides. The translation product of the coding region is a peptide of 355 amino acids. The deduced amino acid sequence of this gene, with a signal peptide (from aa 1-19), a propeptide (20-178) and a mature peptide (from aa 179-335) is shown in Figs. 6 and 7.

### Example 6: Performance of the protease of Thermoascus aurantiacus CGMCC 0670 in a monogastric in vitro digestion model

The AP025 protease was expressed in and excreted from *Aspergillus oryzae.* The purified protease had an A₂₈₀ of 1.15 and an A₂₆₀ of 0.52. The performance of the purified protease was tested in an *in vitro* model simulating the digestion in monogastric animals. In particular, the protease was tested for its ability to improve solubilisation and digestion of maize/-SBM (maize/-Soybean meal) proteins.

The *in vitro* system consisted of 10 flasks in which maize/-SBM substrate was initially incubated with HCl/pepsin - simulating gastric digestion - and subsequently with pancreatin - simulating intestinal digestion. Five of the flasks were dosed with the AP025 protease at the start of the gastric phase whereas the remaining flasks served as blanks. At the end of the intestinal incubation phase samples of *in vitro* digesta were removed and analysed for solubilised and digested protein.

### Outline of in vitro digestion procedure

| **Components added** | **pH** | **Temperature** | **Time course** | **Simulated digestion phase** |
|---|---|---|---|---|
| 10 g maize/-SBM | 3.1 | 40°C | t=0 min | Mixing |
| substrate (6:4), 41 ml HCl (0.105M) | | | | |
| 5 ml HCl (0. 1 05M) / pepsin | 3.1 | 40°C | t=30 min | Gastric digestion |
| (3000 U/g substrate), AP025 protease (100 mg protease enzyme protein/kg diet) | | | | |
| 16 ml H₂O | 3.1 | 40°C | t= 1 hour | Gastric digestion |
| 7 ml NaOH (0,39M) | 6.8 | 40°C | t=1.5 hours | Intestinal digestion |
| 5 ml NaHCO₃ (1 M) / pancreatin | 6.8 | 40°C | t=2 hours | Intestinal digestion |
| (8 mg /g diet) | | | | |
| Terminate incubation | 7.0 | 40°C | t=6 hours | |

### Conditions

| | |
|---|---|
| Substrate: | 4 g SBM, 6 g maize (premixed) |
| pH: | 3.1 stomach step/ 6.8-7.0 intestinal step |
| HCl: | 0.105 M for 1.5 hours (i.e. 30 min HCl-substrate premixing) |
| pepsin: | 3000 U /g diet for 1 hour |
| pancreatin: | 8 mg/g diet for 4 hours |
| temperature: | 40°C. |
| Replicates: | 5 |

### Solutions

0,39 M NaOH
0.105 M HCl
0.105 M HCl containing 6000 U pepsin per 5 ml
1 M NaHCO₃ containing 16 mg pancreatin per ml
125 mM NaAc-buffer, pH 6.0

### Enzyme protein determinations

The amount of protease enzyme protein is calculated on the basis of the A₂₈₀ values and the amino acid sequences (amino acid compositions) using the principles outlined in S.C.Gill & P.H. von Hippel, Analytical Biochemistry 182, 319-326, (1989).

### Experimental procedure for in vitro model

The experimental procedure was according to the above outline. pH was measured at time 1, 2.5, and 5.5 hours. Incubations were terminated after 6 hours and samples of 30 ml were removed and placed on ice before centrifugation (10000 x g, 10 min, 4°C). Supernatants were removed and stored at -20°C.

### Analysis

All samples were analysed for % Degree of Hydrolysis (DH) with the OPA method as well as content of solubilised and digested protein using gelfiltration.

### DH determination by the OPA-method

The degree of hydrolysis (DH) of protein in different samples was determined using an semi-automated micro titer plate based colorimetric method (Nielsen,P.M.; Petersen,D.; Dambmann,C. Improved method for determining food protein degree of hydrolysis. J.Food Sci. 2001, 66, 642-646). The OPA reagent was prepared as follows: 7.620 g di-Na tetraborate decahydrate and 200 mg sodiumdodecyl sulphate (SDS) were dissolved in 150 ml deionised water. The reagents were completely dissolved before continuing. 160 mg o-phthal-dialdehyde 97% (OPA) was dissolved in 4 ml ethanol. The OPA solution was transferred quantitatively to the above-mentioned solution by rinsing with deionised water. 176 mg dithiothreitol 99% (DTT) was added to the solution that was made up to 200 ml with deionised water. A serine standard (0.9516 meqv/l) was prepared by solubilising 50 mg serine (Merck, Germany) in 500 ml deionized water.

The sample solution was prepared by diluting each sample to an absorbance (280 nm) of about 0.5. Generally, supernatants were diluted (100 x) using an automated Tecan dilution station (Männedorf, Switzerland). All other spectrophotometer readings were performed at 340 nm using deionized water as the control. 25 µl of sample, standard and blind was dispensed into a microtiter plate. The micro-titer plate was inserted into an iEMS MF reader (Labsystems, Finland) and 200µl of OPA reagent was automatically dispensed. Plates were shaken (2 min; 700 rpm) before measuring absorbance. Finally, the DH was calculated. Eightfold determination of all samples was carried out.

### Estimation of solubilised and digested protein

The content of solubilised protein in supernatants from *in vitro* digested samples was estimated by quantifying crude protein (CP) using gel filtration HPLC. Supernatants were thawed, filtered through 0.45 µm polycarbonate filters and diluted (1:50, v/v) with H₂O. Diluted samples were chromatographed by HPLC using a Superdex Peptide PE (7.5 x 300 mm) gelfiltration column (Global). The eluent used for isocratic elution was 50 mM sodium phosphate buffer (pH 7.0) containing 150 mM NaCl. The total volume of eluent per run was 26 ml and the flow rate was 0.4 ml/min. Elution profiles were recorded at 214 nm and the total area under the profiles was determined by integration. Elution profiles were recorded at 214 nm and the total area under the profiles was determined by integration. To estimate protein content from integrated areas, a calibration curve (R²=0.9993) was made from a dilution series of an *in vitro* digested reference maize/-SBM sample with known total protein content. The protein determination in this reference sample was carried out by the Kjeldahl method (determination of % nitrogen; A.O.A.C. (1984) Official Methods of Analysis 14th ed., Washington DC).

The content of digested protein was estimated by integrating the chromatogram area corresponding to peptides and amino acids having a molecular mass of 1500 dalton or below (Savoie,L.; Gauthier,S.F. Dialysis Cell For The In-vitro Measurement Of Protein Digestibility. J. Food Sci. 1986, 51, 494-498; Babinszky,L.; Van,D.M.J.M.; Boer,H.; Den,H.L.A. An In-vitro Method for Prediction of The Digestible Crude Protein Content In Pig Feeds. J. Sci. Food Agr. 1990, 50, 173-178; Boisen,S.; Eggum,B.O. Critical Evaluation of In-vitro Methods for Estimating Digestibility in Simple-Stomach Animals. Nutrition Research Reviews 1991, 4, 141-162). To determine the 1500 dalton dividing line, the gel fitration column was calibrated using cytochrome C, aprotinin, gastrin I, and substance P as molecular mass standards.

### Results

The results shown in Tables 1 and 2 below indicate that the AP025 protease increased digestible protein significantly, whereas DH and soluble protein was numerically increased compared to blank.

When tested in the intestinal part of this model only, the AP025 protease (in the same dosage of 100 mg enzyme protein per kg substrate) significantly increased the level of both solubilised and digested protein (103.0 and 103.1%, respectively, relative to the blank), whereas the DH was not increased (96.5% relative to blank).

**Table 1**

| Degree of Hydrolysis (DH), absolute and relative values | | | | | |
|---|---|---|---|---|---|
| Sample | n | Of total protein | | Relative to blank | |
| | | %DH | SD | %DH | %CV |
| Blank | 5 | 26.6 ^{ab} | 0.8 | 100.0 ^{ab} | 2.9 |
| AP025 | 5 | 27.6 ^{b} | 1.4 | 103.5 ^{b} | 5.0 |

| | | | | | |
|---|---|---|---|---|---|
| Different letters within the same column indicate significant differences (1-way ANOVA, Tukey-Kramer test, P<0.05). SD = Standard Deviation. %CV = Coefficient of Variance = (SD/mean value) × 100% | | | | | |

**Table 2**

| Solubilised and digested crude protein measured by AKTA HPLC. | | | | | | | | | | | | | |
|---|---|---|---|---|---|---|---|---|---|---|---|---|---|
| Sample | n | Of total protein | | | | | | Relative to blank | | | | | |
| | | % dig. CP | | SD | % sol. CP | | SD | % dig. CP | | CV% | % sol. CP | | CV% |
| Blank | 5 | 61.5 | ^{a} | 1.6 | 92.2 | ^{a} | 0.7 | 100.0 | ^{a} | 2.6 | 100.0 | ^{a} | 0.7 |
| AP025 | 5 | 64.1 | ^{a} | 0.8 | 93.5 | ^{ab} | 1.0 | 104.2 | ^{b} | 1.2 | 101.4 | ^{ab} | 1.1 |

| | | | | | | | | | | | | | |
|---|---|---|---|---|---|---|---|---|---|---|---|---|---|
| Different letters within the same column indicate significant differences (1-way ANOVA, Tukey-Kramer test, P<0.05). SD = Standard Deviation. %CV = Coefficient of Variance = (SD/mean value) x 100% | | | | | | | | | | | | | |

### Example 7: Performance of the protease of Thermoascus aurantiacus CGMCC 0670 in an aquaculture in vitro digestion model

The same AP025 protease preparation as described in Example 6 was also tested in an aquaculture *in vitro* model simulating digestion in cold-water fish. In comparison to the monogastric model, the aquaculture system is run at slightly different pH-values (pH 3.3 in the gastric phase and pH 7.2 - 7.8 in the intestinal phase). Incubation temperature is lower (15°C instead of 40°C) and incubation periods are longer (outlined below).10 flasks containing extruded SBM as the substrate were initially incubated with HCl/pepsin - simulating gastric digestion - and subsequently with pancreatin - simulating intestinal digestion. Five of the flasks were dosed with the AP025 protease at the start of the gastric phase whereas the remaining flasks served as blanks. At the end of the intestinal incubation phase samples of *in vitro* digesta were removed and analysed for solubilised and digested protein.

### Outline of aquaculture in vitro procedure simulating digestion in cold-water fish

| **Components added** | **pH** | **Temperature** | **Time course** | **Simulated digestion phase** |
|---|---|---|---|---|
| 10 g extruded SBM, 46 ml HCl (0.18 M)/pepsin (3000 U/g extruded SBM), AP025 protease (100 mg protease enzyme protein/kg extruded SBM), 16 ml H₂O | 3.3 | 15°C | t=0 min | Gastric digestion |
| 7 ml NaOH (1.10M) | 7.2 | 15°C | t=6 hours | Intestinal digestion |
| 5 ml NaHCO₃ (1M) / pancreatin | 7.2 | 15°C | t=7 hours | Intestinal digestion |
| (8 mg /g extruded SBM) | | | | |
| Terminate incubation | 7.8 | 15°C | t=24 hours | |

### Conditions

| | |
|---|---|
| Substrate: | 10 g extruded SBM |
| pH: | 3.3 stomach step/ 7.2 - 7.8 intestinal step |
| HCl: | 0.18 M for 6 hours |
| pepsin: | 3000 U /g extruded for 6 hours |
| pancreatin: | 8 mg/g extruded SBM for 18 hours. |
| Temperature: | 15°C. |
| Replicates: | 5 |

### Solutions

1.10 M NaOH
0.18 M HCl containing 652 U pepsin per ml
1 M NaHCO₃ containing 16 mg pancreatin per ml
125 mM NaAc-buffer, pH 6.0

### Experimental procedure for the aquaculture in vitro digestion model

The experimental procedure was carried out as outlined above. pH was measured after 1, 5, 8 and 23 hours. Incubations were terminated after 24 hours and samples of 30 ml were removed and placed on ice before centrifugation (10000 x g, 10 min, 4°C). Supernatants were removed and stored at -20°C.

### Analysis

All supernatants were analysed with the OPA method (% degree of hydrolysis) and Akta HPLC for solubilised and digested protein (see Example 6).

### Results

The results shown in Tables 3 and 4 below indicate that the AP025 protease significantly increased protein solubility, protein digestibility and DH, relative to the blank.

**Table 3**

| OPA results in % degree of hydrolysis. | | | | | |
|---|---|---|---|---|---|
| Sample | n | Of total protein | | Relative to blank | |
| | | %DH | SD | %DH | %CV |
| Blank | 5 | 20.4 | 1.3^{a} | 100.0 | 6.4^{a} |
| AP025 | 5 | 23.0 | 0.2^{bc} | 112.8 | 1.1^{bc} |

| | | | | | |
|---|---|---|---|---|---|
| Different superscripts within one column indicate significant differences (1-way ANOVA, Tukey-Kramer test, P<0.05). | | | | | |

**Table 4**

| Results on digested (%CPdig) and solubilised (%CPsol) crude protein analysed by Akta HPLC. | | | | | | | | | |
|---|---|---|---|---|---|---|---|---|---|
| Sample | n | Of total protein | | | | Relative to blank | | | |
| | | % dig. CP | SD | % sol. CP | SD | % dig. CP | CV% | % sol. CP | CV% |
| Blank | 5 | 45.9 ^{a} | 2.9 | 82.7 ^{a} | 5.6 | 100.0 ^{a} | 6.2 | 100.0 ^{a} | 6.8 |
| AP025 | 5 | 51.8 ^{bc} | 0.8 | 90.9 ^{bc} | 2.4 | 112.9 ^{bc} | 1.6 | 109.9 ^{bc} | 2.6 |

| | | | | | | | | | |
|---|---|---|---|---|---|---|---|---|---|
| Different superscripts indicate significant differences. (ANOVA - Tukey-Kramer test, P<0.05). | | | | | | | | | |

### Deposit of Biological Material

The following biological material has been deposited under the terms of the Budapest Treaty with DSMZ (DSMZ-Deutsche Sammlung von Mikroorganismen und Zellkulturen GmbH, Mascheroder Weg 1b, D-38124 Braunschweig, Germany), and CGMCC (the China General Microbiological Culture Collection Center, Institute of Microbiology, Chinese Academy of Sciences, Haidian, Beijing 100080, China); and given the following accession numbers:

| Deposit | Accession Number | Date of Deposit |
|---|---|---|
| *Escherichia coli* | DSM 14652 | 2001-11-29 |
| *Thermoascus aurantiacus* | CGMCC No. 0670 | 2001-12-27 |

The *Escherichia* coli strain harbours a plasmid containing the nucleic acid sequence of the protease of *Thermoascus aurantiacus* (i.e. SEQ ID NO: 1 encoding SEQ ID NO:2).

These strains have been deposited under conditions that assure that access to the cultures will be available during the pendency of this patent application to one determined by the Commissioner of Patents and Trademarks to be entitled thereto under 37 C.F.R. §1.14 and 35 U.S.C. §122. The deposits represent a substantially pure culture of the deposited strains. The deposits are available as required by foreign patent laws in countries wherein counterparts of the subject application, or its progeny are filed. However, it should be understood that the availability of a deposit does not constitute a license to practice the subject invention in derogation of patent rights granted by governmental action.

*Thermoascus aurantiacus* strain no. CGMCC 0670 was isolated from a soil sample collected on July 21, 1998 in the Yunnan Province, Xishuangbanna, China.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

## Claims

1. An isolated polypeptide having protease activity, selected from the group consisting of:
(a) a polypeptide having an amino acid sequence which has a degree of identity to amino acids -178 to 177, -159 to 177, or +1 to 177 of SEQ ID NO:2 of at least 80%;
(b) a polypeptide which is encoded by a nucleic acid sequence which hybridizes under medium stringency conditions with
(i) the mature protease encoding part of the plasmid contained in *Escherichia coli* DSM 14652,
(ii) nucleotides 25 to 1089, 1 to 1089, 1 to 1344, 25 to 1344, 559 to 1344, or 559 to 1089 of SEQ ID NO:1,
(iii) a subsequence of (i) or (ii) of at least 100 nucleotides, or
(iv) a complementary strand of (i), (ii) or (iii); and
(c) a fragment of (a) or (b) that has protease activity.

2. The polypeptide of claim 1 which is
(i) an EC 3.4.24 metalloendopeptidase;
(ii) a family M metalloprotease as defined at pp. 989-991 of "Handbook of Proteolytic Enzymes" by Barrett et al (eds), Academic Press (1998); and/or
(iii) which has an HEFTH motif.

3. An isolated nucleic acid sequence, which
(a) comprises a nucleic acid sequence which encodes the polypeptide of any one of claims 1-2;
(b) encodes a polypeptide having protease activity, and which hybridizes under medium stringency conditions with
(i) the mature protease encoding part of the plasmid contained in *Escherichia coli* DSM 14652,
(ii) nucleotides 25 to 1089, 1 to 1089, 1 to 1344, 25 to 1344, 559 to 1344, or 559 to 1089 of SEQ ID NO:1;
(iii) a subsequence of (i) or (ii) of at least 100 nucleotides; and/or
(iv) a complementary strand of (i), (ii), or (iii); and/or
(c) encodes a polypeptide having protease activity, and which has a degree of identity to nucleotides 25 to 1089, 1 to 1089, 1 to 1344, 25 to 1344, 559 to 1344, or 559 to 1089 of SEQ ID NO:1 of at least 70%.

4. A nucleic acid construct comprising the nucleic acid sequence of claim 3 operably linked to one or more control sequences that direct the production of the polypeptide in a suitable expression host.

5. A recombinant expression vector comprising the nucleic acid construct of claim 4.

6. A recombinant host cell comprising the nucleic acid construct of claim 4 or the vector of claim 5.

7. A method for producing a polypeptide of any one of claims 1-2, the method comprising (a) cultivating a recombinant host cell of claim 6 to produce a supernatant comprising the polypeptide; and (b) recovering the polypeptide.

8. A method for producing a polypeptide of any one of claims 1 or 2, the method comprising (a) cultivating a strain of the genus *Thermoascus;* and (b) recovering the polypeptide.

9. *Thermoascus aurantiacus* CGMCC No. 0670.

10. Use of the protease of claim 1 or 2
(i) in animal feed; and/or
(ii) in the preparation of a composition for use in animal feed.

11. A method for improving the nutritional value of an animal feed, wherein the protease of claim 1 or 2 is added to the feed.

12. An animal feed additive comprising
(a) the protease of claim 1 or 2; and
(b) at least one fat-soluble vitamin, and/or
(c) at least one water-soluble vitamin, and/or
(d) at least one trace mineral, and/or
(e) at least one macro mineral;
the additive optionally further comprising phytase, xylanase, galactanase, and/or beta-glucanase.

13. An animal feed composition having a crude protein content of 50 to 800 g/kg and comprising the protease of claim 1 or 2.

14. A method for the treatment of vegetable proteins, comprising the step of adding the protease of claim 1 or 2 to at least one vegetable protein or protein source.

15. The method of claim 14, wherein soybean is included amongst the at least one vegetable protein source.

## Patentansprüche

1. Isoliertes Polypeptid mit Proteaseaktivität, ausgewählt aus der Gruppe, bestehend aus
(a) einem Polypeptid mit einer Aminosäuresequenz, die einen Grad an Identität zu Aminosäuren -178 bis 177, -159 bis 177, oder +1 bis 177 von SEQ ID NO: 2 von mindestens 80 % hat;
(b) einem Polypeptid, das durch eine Nukleinsäuresequenz kodiert ist, die unter mittleren Stringenzbedingungen hybridisiert mit
(i) dem die reife Protease kodierenden Teil des Plasmids, das in *Escherichia coli* DSM 14652 enthalten ist,
(ii) Nukleotiden 25 bis 1089, 1 bis 1089, 1 bis 1344, 25 bis 1344, 559 bis 1344 oder 559 bis 1089 von SEQ ID NO: 1,
(iii) einer Untersequenz von (i) oder (ii) von mindestens 100 Nukleotiden, oder
(iv) einem komplementären Strang von (i), (ii) oder (iii); und
(c) einem Fragment von (a) oder (b), das Proteaseaktivität hat.

2. Polypeptid nach Anspruch 1, das
(i) eine EC 3.4.24 Metalloendopeptidase ist;
(ii) eine Metalloprotease der M-Familie ist, wie definiert auf S. 989-991 von "Handbook of Proteolytic Enyzmes" von Barrett et al., (Hrsg.), Academic Press (1998); und/oder
(iii) ein HEFTH-Motiv hat.

3. Isolierte Nukleinsäuresequenz, die
(a) eine Nukleinsäuresequenz umfasst, die das Polypeptid nach einem beliebigen der Ansprüche 1-2 kodiert;
(b) ein Polypeptid mit Proteaseaktivität kodiert, und die unter mittleren Stringenzbedingungen hybridisiert mit
(i) dem die reife Protease kodierenden Teil des Plasmids, das in *Escherichia coli* DSM 14652 enthalten ist,
(ii) Nukleotiden 25 bis 1089, 1 bis 1089, 1 bis 1344, 25 bis 1344, 559 bis 1344 oder 559 bis 1089 von SEQ ID NO: 1,
(iii) einer Untersequenz von (i) oder (ii) von mindestens 100 Nukleotiden; und/oder
(iv) einem komplementären Strang von (i), (ii) oder (iii); und/oder
(c) ein Polypeptid mit Proteaseaktivität kodiert, und die einem Grad an Identität zu Nukleotiden 25 bis 1089, 1 bis 1089, 1 bis 1344, 25 bis 1344, 559 bis 1344 oder 559 bis 1089 von SEQ ID NO: 1 von mindestens 70 % hat.

4. Nukleinsäurekonstrukt, umfassend die Nukleinsäuresequenz gemäß Anspruch 3 funktionsfähig verknüpft mit einer oder mehreren Kontrollsequenzen, die die Herstellung des Polypeptids in einem geeigneten Expressionswirt steuert/steuern.

5. Rekombinanter Expressionsvektor, umfassend das Nukleinsäurekonstrukt gemäß Anspruch 4.

6. Rekombinante Wirtszelle, umfassend das Nukleinsäurekonstrukt gemäß Anspruch 4 oder den Vektor gemäß Anspruch 5.

7. Verfahren zum Herstellen eines Polypeptids nach einem beliebigen der Ansprüche 1 bis 2, wobei das Verfahren (a) das Kultivieren einer rekombinanten Wirtszelle gemäß Anspruch 6 zur Herstellung eines Überstandes, umfassend das Polypeptid; und (b) das Gewinnen des Polypeptids umfasst.

8. Verfahren zum Herstellen eines Polypeptids nach einem beliebigen der Ansprüche 1 oder 2, wobei das Verfahren (a) das Kultivieren eines Stammes der Gattung *Thermoascus;* und (b) Gewinnen des Polypeptids umfasst.

9. *Thermoascus aurantiacus* CGMCC Nr. 0670.

10. Verwendung der Protease gemäß Anspruch 1 oder 2
(i) in Tierfutter; und/oder
(ii) bei der Zubereitung einer Zusammensetzung zur Verwendung in Tierfutter.

11. Verfahren zum Verbessern des Nährwertes eines Tierfutters, wobei die Protease gemäß Anspruch 1 oder 2 dem Futter hinzugefügt wird.

12. Tierfutteradditiv, umfassend
(a) die Protease gemäß Anspruch 1 oder 2; und
(b) mindestens ein fettlösliches Vitamin, und/oder
(c) mindestens ein wasserlösliches Vitamin, und/oder
(d) mindestens ein Spurenmineral, und/oder
(e) mindestens ein Makromineral;
wobei das Additiv wahlweise Phytase, Xylanase, Galactanase und/oder beta-Glucanase umfasst.

13. Tierfutterzusammensetzung mit einem Rohproteingehalt von 50 bis 800 g/kg und umfassend die Protease gemäß Anspruch 1 oder 2.

14. Verfahren zur Behandlung von Pflanzenproteinen, umfassend den Schritt des Hinzufügens der Protease gemäß Anspruch 1 oder 2 zu mindestens einem Pflanzenprotein oder einer Pflanzenquelle.

15. Verfahren nach Anspruch 14, wobei die mindestens eine Pflanzenproteinquelle Sojabohne einschließt.

## Revendications

1. Polypeptide isolé possédant une activité de protéase, choisi parmi le groupe constitué par :
(a) un polypeptide possédant une séquence d'acides aminés qui possède un degré d'identité des acides aminés -178 à 177, -159 à 177 ou +1 à 177 de la SEQ ID NO: 2 s'élevant à au moins 80 % ;
(b) un polypeptide qui est encodé par une séquence d'acides nucléiques qui s'hybride dans des conditions de stringence moyenne avec
(i) la protéase mature encodant une partie du plasmide contenu dans Escherichia coli DSM 14652,
(ii) les nucléotides 25 à 1089, 1 à 1089, 1 à 1344, 25 à 1344, 559 à 1344, ou 559 à 1089 de la SEQ ID NO: 1,
(iii) une sous-séquence de (i) ou de (ii) contenant au moins 100 nucléotides, ou
(iv) un brin complémentaire de (i), de (ii) ou de (iii) ; et
(c) un fragment de (a) ou de (b) qui possède une activité de protéase.

2. Polypeptide selon la revendication 1, à savoir
(i) une métalloendopeptidase EC 3.4.24 ;
(ii) une famille de métalloprotéases M comme défini aux pages 989-991 du « Handbook of Proteolytic Enzymes » par Barrett et al (eds), Academic Press (1998) ; et/ou
(iii) un polypeptide qui possède un motif HEFTH.

3. Séquence isolée d'acides nucléiques, qui
(a) comprend une séquence d'acides nucléiques qui encode le polypeptide selon l'une quelconque des revendications 1-2 ;
(b) encode un polypeptide possédant une activité de protéase et qui s'hybride dans des conditions de stringence moyenne, avec
(i) la protéase mature encodant une partie du plasmide contenu dans Escherichia coli DSM 14652,
(ii) les nucléotides 25 à 1089, 1 à 1089, 1 à 1344, 25 à 1344, 559 à 1344, ou 559 à 1089 de la SEQ ID NO: 1,
(iii) une sous-séquence de (i) ou de (ii) contenant au moins 100 nucléotides, et/ou
(iv) un brin complémentaire de (i), de (ii) ou de (iii) ; et/ou
(c) encode un polypeptide possédant une activité de protéase, et qui possède un degré d'identité par rapport aux nucléotides 25 à 1089, 1 à 1089, 1 à 1344, 25 à 1344, 559 à 1344, ou 559 à 1089 de la SEQ ID NO: 1 s'élevant à au moins 70 %.

4. Construction d'acides nucléiques comprenant la séquence d'acides nucléiques selon la revendication 3, liée de manière opérante à une ou plusieurs séquences de commande qui dirigent la production du polypeptide dans un autre d'expression approprié.

5. Vecteur d'expression recombinant comprenant la construction d'acides nucléiques selon la revendication 4.

6. Cellule hôte recombinante comprenant la construction d'acides nucléiques selon la revendication 4 ou le vecteur selon la revendication 5.

7. Procédé de production d'un polypeptide selon l'une quelconque des revendications 1-2, le procédé comprenant (a) la culture d'une cellule hôte recombinante selon la revendication 6 pour obtenir un produit surnageant comprenant le polypeptide ; et (b) récupérer le polypeptide.

8. Procédé de production d'un polypeptide selon l'une quelconque des revendications 1 ou 2, le procédé comprenant (a) la culture d'une souche du genre *Thermoascus ;* et (b) la récupération du polypeptide.

9. *Thermoascus aurantiacus* CGMCC No. 0670.

10. Utilisation de la protéase selon la revendication 1 ou 2
(i) dans un aliment pour animaux ; et/ou
(ii) dans la préparation d'une composition à utiliser dans un aliment pour animaux.

11. Procédé pour améliorer la valeur nutritionnelle d'un aliment pour animaux, dans lequel la protéase selon la revendication 1 ou 2 est ajoutée à l'aliment pour animaux.

12. Additif d'aliment pour animaux, comprenant :
(a) la protéase selon la revendication 1 ou 2 ; et
(b) au moins une vitamine soluble dans les graisses ; et/ou
(c) au moins une vitamine soluble dans l'eau ; et/ou
(d) au moins un oligo-élément ; et/ou
(e) au moins un macro-élément ;
l'additif comprenant en outre, de manière facultative, de la phytase, de la xylanase, de la galactanase, et/ou de la bêta-glucanase.

13. Composition d'aliments pour animaux possédant une teneur en protéines brutes de 50 à 800 g/kg et comprenant la protéase selon la revendication 1 ou 2.

14. Procédé de traitement de protéines végétales, comprenant l'étape d'addition de la protéase selon la revendication 1 ou 2 à au moins une protéine végétale ou une source de protéine végétale.

15. Procédé selon la revendication 14, dans lequel le soja est inclus dans ladite au moins une source de protéines végétales.


### SEQUENCE LISTING

<110> Novozymes A/S
<120> Proteases
<130> 10251
<160> 11
<170> PatentIn version 3.1
<210> 1
   <211> 1344
   <212> DNA
   <213> Thermoascus aurantiacus
<220>
   <221> misc_feature
   <222> (25) .. (1089)
   <223> Gene encoding SEQ ID NO:2
<400> 1
   aagtctaccc agtatcctgt caacatgcgg ctcgttgctt ccctaacggc cttggtggcc 60
   ttgtccgtac ctgtctttcc cgctgctgtc aacgtgaagc gtgcttcgtc ctacctggag 120
   atcactctga gccaggtcag caacactctg atcaaggccg tggtccagaa cactggtagc 180
   gacgagttgt ccttcgttca cctgaacttc ttcaaggacc ccgctcctgt caaaaaggta 240
   tcggtctatc gcgatgggtc tgaagtgcag ttcgagggca ttttgagccg ctacaaatcg 300
   actggcctct ctcgtgacgc ctttacttat ctggctcccg gagagtccgt cgaggacgtt 360
   tttgatattg cttcgactta cgatctgacc agcggcggcc ctgtaactat ccgtactgag 420
   ggagttgttc cctacgccac ggctaacagc actgatattg ccggctacat ctcatactcg 480
   tctaatgtgt tgaccattga tgtcgatggc gccgctgctg ccactgtctc caaggcaatc 540
   actcctttgg accgccgcac taggatcagt tcctgctccg gcagcagaca gagcgctctt 600
   actacggctc tcagaaacgc tgcttctctt gccaacgcag ctgccgacgc ggctcagtct 660
   ggatcagctt caaagttcag cgagtacttc aagactactt ctagctctac ccgccagacc 720
   gtggctgcgc gtcttcgggc tgttgcgcgg gaggcatctt cgtcttcttc gggagccacc 780
   acgtactact gcgacgatcc ctacggctac tgttcctcca acgtcctggc ttacaccctg 840
   ccttcataca acataatcgc caactgtgac attttctata cttacctgcc ggctctgacc 900
   agtacctgtc acgctcagga tcaagcgacc actgcccttc acgagttcac ccatgcgcct 960
   ggcgtctaca gccctggcac ggacgacctg gcgtatggct accaggctgc gatgggtctc 1020
   agcagcagcc aggctgtcat gaacgctgac acctacgctc tctatgcgaa tgccatatac 1080
   cttggttgct aagcgcagag cggtccattg gcgagttggt cgcggtccag ctctagctgg 1140
   gatcggccat ggatggtttg agctctgtaa atgacggtcc cgatcttgca gctttgattc 1200
   catctaaacg cgcaggaagg aatattagga tgaggatgtt tctatgagac ggctgtgcgc 1260
   agagttccga cgagtgacgg taactatttt tgccatagct acataatgca tctacaagtt 1320
   atctaaaaaa aaaaaaaaaa aaaa 1344
<210> 2
   <211> 355
   <212> PRT
   <213> Thermoascus aurantiacus
<220>
   <221> SIGNAL
   <222> (1)..(19)
   <223>
<220>
   <221> mat_peptide
   <222> (179)..()
   <223>
<220>
   <221> PROPEP
   <222> (20)..(178)
   <223>
<400> 2
   Met Arg Leu Val  Ala Ser Leu Thr Ala  Leu Val Ala Leu Ser  Val
            -175               -170                -165
   Pro Val Phe Pro  Ala Ala Val Asn Val  Lys Arg Ala Ser Ser  Tyr
            -160               -155                -150
   Leu Glu Ile Thr  Leu Ser Gln Val Ser  Asn Thr Leu Ile Lys  Ala
            -145               -140                -135
   Val Val Gln Asn  Thr Gly Ser Asp Glu  Leu Ser Phe Val His  Leu
            -130               -125                -120
   Asn Phe Phe Lys  Asp Pro Ala Pro Val  Lys Lys Val Ser Val  Tyr
            -115               -110                -105
   Arg Asp Gly Ser  Glu Val Gln Phe Glu Gly Ile Leu Ser Arg Tyr Lys
            -100               -95                -90
   Ser Thr Gly Leu Ser Arg Asp Ala Phe Thr Tyr Leu Ala Pro Gly Glu
         -85               -80               -75
   Ser Val Glu Asp Val Phe Asp Ile Ala Ser Thr Tyr Asp Leu Thr Ser
       -70                 -65                 -60
   Gly Gly Pro Val Thr Ile Arg Thr Glu Gly Val Val Pro Tyr Ala Thr
   -55                 -50                 -45                 -40
   Ala Asn Ser Thr Asp Ile Ala Gly Tyr Ile Ser Tyr Ser Ser Asn Val
                   -35                 -30                 -25
   Leu Thr Ile Asp Val Asp Gly Ala Ala Ala Ala Thr Val Ser Lys Ala
               -20                 -15                 -10
   Ile Thr Pro Leu Asp Arg Arg Thr Arg Ile Ser Ser Cys Ser Gly Ser
           -5                  -1  1               5
   Arg Gln Ser Ala Leu Thr Thr Ala Leu Arg Asn Ala Ala Ser Leu Ala
   10                  15                  20                  25
   Asn Ala Ala Ala Asp Ala Ala Gln Ser Gly Ser Ala Ser Lys Phe Ser
                   30                  35                  40
   Glu Tyr Phe Lys Thr Thr Ser Ser Ser Thr Arg Gln Thr Val Ala Ala
               45                  50                  55
   Arg Leu Arg Ala Val Ala Arg Glu Ala Ser Ser Ser Ser Ser Gly Ala
           60                  65                  70
   Thr Thr Tyr Tyr Cys Asp Asp Pro Tyr Gly Tyr Cys Ser Ser Asn Val
       75                  80                  85
   Leu Ala Tyr Thr Leu Pro Ser Tyr Asn Ile Ile Ala Asn Cys Asp Ile
   90                  95                  100                 105
   Phe Tyr Thr Tyr Leu Pro Ala Leu Thr Ser Thr Cys His Ala Gln Asp
                   110                 115                 120
   Gln Ala Thr Thr Ala Leu His Glu Phe Thr His Ala Pro Gly Val Tyr
               125                 130                 135
   Ser Pro Gly Thr Asp Asp Leu Ala Tyr Gly Tyr Gln Ala Ala Met Gly
           140                 145                 150
   Leu Ser Ser Ser Gln Ala Val Met Asn Ala Asp Thr Tyr Ala Leu Tyr
       155                 160                 165
   Ala Asn Ala Ile Tyr Leu Gly Cys
   170 175
<210> 3
   <211> 20
   <212> PRT
   <213> Thermoascus aurantiacus
<220>
   <221> MISC_FEATURE
   <223> N-terminal amino acid sequence
<400> 3
   Gln Arg Ile Ser Ser Cys Ser Gly Ser Arg Gln Ser Ala Leu Thr Thr
   1 5 10 15
   Ala Leu Arg Asn
   20
<210> 4
   <211> 20
   <212> DNA
   <213> Artificial
<220>
   <223> Degenerate primer
<220>
   <221> misc_feature
   <222> (3)..(3)
   <223> N in position 3 and 18 means A or G
<220>
   <221> misc_feature
   <222> (18)..(18)
   <223> N in position 3 and 18 means A or G
<220>
   <221> misc_feature
   <222> (6)..(6)
   <223> M in position 6 means T, C, A or G
<220>
   <221> misc_feature
   <222> (9)..(9)
   <223> M in position 9 means T, C, A or G
<220>
   <221> misc_feature
   <222> (12)..(12)
   <223> M in position 12 means T, C, A or G
<220>
   <221> misc_feature
   <222> (15)..(15)
   <223> K in position 15 means T or C
<400> 4
   cantcmgcmc tmackacngc 20
<210> 5
   <211> 20
   <212> DNA
   <213> Artificial
<220>
   <223> Degenerate primer
<220>
   <221> misc_feature
   <222> (3)..(3)
   <223> N in position 3 means A or G
<220>
   <221> misc_feature
   <222> (6)..(6)
   <223> K in position 6 means T or C
<220>
   <221> misc_feature
   <222> (9)..(9)
   <223> M in position 9 means T, C, A or G
<220>
   <221> misc_feature
   <222> (12)..(12)
   <223> M in position 12 means T, C, A or G
<220>
   <221> misc_feature
   <222> (15)..(15)
   <223> N in position 15 means A or G
<220>
   <221> misc_feature
   <222> (18)..(18)
   <223> N in position 18 means A or G
<400> 5
   canagkgcmc tmacnacngc 20
<210> 6
   <211> 20
   <212> DNA
   <213> Artificial
<220>
   <223> Primer
<400> 6
   aaggtatatg gcattcgcat 20
<210> 7
   <211> 18
   <212> DNA
   <213> Artificial
<220>
   <223> Primer
<400> 7
   gcagcctggt agccatac 18
<210> 8
   <211> 19
   <212> DNA
   <213> Artificial
<220>
   <223> Primer
<400> 8
   ttgatcctga gcgtgacag 19
<210> 9
   <211> 20
   <212> DNA
   <213> Artificial
<220>
   <223> Primer
<400> 9
   aagtctaccc agtatcctgt 20
<210> 10
   <211> 1267
   <212> DNA
   <213> Aspergillus oryzae
<220>
   <221> CDS
   <222> (1133)..(1195)
   <223>
<220>
   <221> mat_peptide
   <222> (71)..()
   <223>
<220>
   <221> CDS
   <222> (2)..(70)
   <223>
<220>
   <221> CDS
   <222> (1199)..(1267)
   <223>
<220>
   <221> CDS
   <222> (71)..(1129)
   <223>
<400> 10
   c cac gcg tcc gcg gac gcg tgg gcg agt tca aaa gcc att tcc agt act 49
   His Ala Ser Ala Asp Ala Trp Ala Ser Ser Lys Ala Ile Ser Ser Thr
   -20 -15 -10
   tca agt cct tca agc ttc aag atg cgt ttc att tct gtc tcc tct ctt 97
   Ser Ser Pro Ser Ser Phe Lys Met Arg Phe Ile Ser Val Ser Ser Leu
   -5 -1 1 5
   ctt tta gcc ctg gca ccg gct ctc aat gcc gtt cct gta gag gtt gcc 145
   Leu Leu Ala Leu Ala Pro Ala Leu Asn Ala Val Pro Val Glu Val Ala
   10 15 20 25
   ggt agt gcc caa gga ctt gat gtg act ctt agc cag gtg gga aat act 193
   Gly Ser Ala Gln Gly Leu Asp Val Thr Leu Ser Gln Val Gly Asn Thr
   30 35 40
   cgg atc aag gcc gtg gta aag aac act ggc agc gag gat gtc acc ttt 241
   Arg Ile Lys Ala Val Val Lys Asn Thr Gly Ser Glu Asp Val Thr Phe
   45 50 55
   gtg cac ctc aac ttc ttc aag gat gcc gct ccg gtg cag aag gtg tct 289
   Val His Leu Asn Phe Phe Lys Asp Ala Ala Pro Val Gln Lys Val Ser
   60 65 70
   ctg ttc cgc aat gcg acc gag gtt caa ttc cag ggt atc aag cag cgt 337
   Leu Phe Arg Asn Ala Thr Glu Val Gln Phe Gln Gly Ile Lys Gln Arg
   75 80 85
   ctt atc acc gaa ggc ttg tcc gat gat gct ttg aca act ctt gcc cct 385
   Leu Ile Thr Glu Gly Leu Ser Asp Asp Ala Leu Thr Thr Leu Ala Pro
   90 95 100 105
   ggt gct act atc gag gac gag ttc gat atc gca agt act agt gac ctg 433
   Gly Ala Thr Ile Glu Asp Glu Phe Asp Ile Ala Ser Thr Ser Asp Leu
   110 115 120
   tcc gag ggt ggt acc atc acg atc aac agc aac ggt tta gta cct att 481
   Ser Glu Gly Gly Thr Ile Thr Ile Asn Ser Asn Gly Leu Val Pro Ile
   125 130 135
   acc acc gat aac aag gtc act gga tac att cca ttc acc tcg aac gag 529
   Thr Thr Asp Asn Lys Val Thr Gly Tyr Ile Pro Phe Thr Ser Asn Glu
   140 145 150
   ctc tcc att gat gta gat gca gct gag gcc gcg agt gtt act caa gca      577
   Leu Ser Ile Asp Val Asp Ala Ala Glu Ala Ala Ser Val Thr Gln Ala
       155                 160                 165
   gtt aag atc ctg gaa cgc cgc acc aag gtc act tcc tgc tct ggc agc      625
   Val Lys Ile Leu Glu Arg Arg Thr Lys Val Thr Ser Cys Ser Gly Ser
   170                 175                 180                 185
   aga ttg tcg gcc ctt cag act gct ctg aga aac aca gtc tct ttg gca      673
   Arg Leu Ser Ala Leu Gln Thr Ala Leu Arg Asn Thr Val Ser Leu Ala
                   190                 195                 200
   cgt gca gct gct act gcc gcg cag tcg gga tct tcc tcc cgt ttc cag      721
   Arg Ala Ala Ala Thr Ala Ala Gln Ser Gly Ser Ser Ser Arg Phe Gln
               205                 210                 215
   gag tat ttc aag acg aca tcc agc tcc acc cgt agc acg gtt gct gct      769
   Glu Tyr Phe Lys Thr Thr Ser Ser Ser Thr Arg Ser Thr Val Ala Ala
           220                 225                 230
   cgc ctg aac gcc gtt gct aac gag gcc gcg tcg acc tct tcg gga agt      817
   Arg Leu Asn Ala Val Ala Asn Glu Ala Ala Ser Thr Ser Ser Gly Ser
       235                 240                 245
   acc acg tac tac tgc agc gac gtg tat gga tac tgc agc tcc aac gtg      865
   Thr Thr Tyr Tyr Cys Ser Asp Val Tyr Gly Tyr Cys Ser Ser Asn Val
   250                 255                 260                 265
   ctt gcg tat acc ctt ccg tct tat aac atc atc gcc aac tgc gac ctc      913
   Leu Ala Tyr Thr Leu Pro Ser Tyr Asn Ile Ile Ala Asn Cys Asp Leu
                   270                 275                 280
   tac tat tcc tat ctt ccg gca ctg act agc acc tgc cat gct cag gac      961
   Tyr Tyr Ser Tyr Leu Pro Ala Leu Thr Ser Thr Cys His Ala Gln Asp
               285                 290                 295
   cag gcc acc acc acc ctg cat gag ttc act cac gcc ccc ggt gtg tac     1009
   Gln Ala Thr Thr Thr Leu His Glu Phe Thr His Ala Pro Gly Val Tyr
           300                 305                 310
   agc cct ggc act gac gac ctt ggc tat gga tac tcg gct gcc acc gcc     1057
   Ser Pro Gly Thr Asp Asp Leu Gly Tyr Gly Tyr Ser Ala Ala Thr Ala
       315                 320                 325
   ttg agt gcc agt cag gct ctg ctg aat gcc gac acc tat gcc ttg ttt     1105
   Leu Ser Ala Ser Gln Ala Leu Leu Asn Ala Asp Thr Tyr Ala Leu Phe
   330                 335                 340                 345
   gcc aac gct gtc aac ctc aac tgt tag acg cac gta ctt gca atc ggc     1153
   Ala Asn Ala Val Asn Leu Asn Cys     Thr His Val Leu Ala Ile Gly
                   350                 355                 360
   aat ttg gaa gga gta ctt cct gca agg tgt att gga aag ctg tag agt     1201
   Asn Leu Glu Gly Val Leu Pro Ala Arg Cys Ile Gly Lys Leu     Ser
                   365                 370                 375
   gaa tat atg att ggt gct gat ggc att aaa gtt tat aca atg aga tac     1249
   Glu Tyr Met Ile Gly Ala Asp Gly Ile Lys Val Tyr Thr Met Arg Tyr
                   380                 385                 390
   aat gga gcc att cac ctg                                              1267
   Asn Gly Ala Ile His Leu
               395
<210> 11
   <211> 420
   <212> PRT
   <213> Aspergillus oryzae
<400> 11
   His Ala Ser Ala Asp Ala Trp Ala Ser Ser Lys Ala Ile Ser Ser Thr
            -20                -15                -10
   Ser Ser Pro Ser Ser Phe Lys Met Arg Phe Ile Ser Val Ser Ser Leu
          -5            -1   1              5
   Leu Leu Ala Leu Ala Pro Ala Leu Asn Ala Val Pro Val Glu Val Ala
   10                15                20                25
   Gly Ser Ala Gln Gly Leu Asp Val Thr Leu Ser Gln Val Gly Asn Thr
                 30                35                40
   Arg Ile Lys Ala Val Val Lys Asn Thr Gly Ser Glu Asp Val Thr Phe
            45                50                55
   Val His Leu Asn Phe Phe Lys Asp Ala Ala Pro Val Gln Lys Val Ser
          60                65                70
   Leu Phe Arg Asn Ala Thr Glu Val Gln Phe Gln Gly Ile Lys Gln Arg
       75                80                85
   Leu Ile Thr Glu Gly Leu Ser Asp Asp Ala Leu Thr Thr Leu Ala Pro
   90                95                100               105
   Gly Ala Thr Ile Glu Asp Glu Phe Asp Ile Ala Ser Thr Ser Asp Leu
                 110               115               120
   Ser Glu Gly Gly Thr Ile Thr Ile Asn Ser Asn Gly Leu Val Pro Ile
            125               130               135
   Thr Thr Asp Asn Lys Val Thr Gly Tyr Ile Pro Phe Thr Ser Asn Glu
          140               145               150
   Leu Ser Ile Asp Val Asp Ala Ala Glu Ala Ala Ser Val Thr Gln Ala
       155               160               165
   Val Lys Ile Leu Glu Arg Arg Thr Lys Val Thr Ser Cys Ser Gly Ser
   170                 175                 180                 185
   Arg Leu Ser Ala Leu Gln Thr Ala Leu Arg Asn Thr Val Ser Leu Ala
                   190                 195                 200
   Arg Ala Ala Ala Thr Ala Ala Gln Ser Gly Ser Ser Ser Arg Phe Gln
               205                 210                 215
   Glu Tyr Phe Lys Thr Thr Ser Ser Ser Thr Arg Ser Thr Val Ala Ala
           220                 225                 230
   Arg Leu Asn Ala Val Ala Asn Glu Ala Ala Ser Thr Ser Ser Gly Ser
       235                 240                 245
   Thr Thr Tyr Tyr Cys Ser Asp Val Tyr Gly Tyr Cys Ser Ser Asn Val
   250                 255                 260                 265
   Leu Ala Tyr Thr Leu Pro Ser Tyr Asn Ile Ile Ala Asn Cys Asp Leu
                   270                 275                 280
   Tyr Tyr Ser Tyr Leu Pro Ala Leu Thr Ser Thr Cys His Ala Gln Asp
               285                 290                 295
   Gln Ala Thr Thr Thr Leu His Glu Phe Thr His Ala Pro Gly Val Tyr
           300                 305                 310
   Ser Pro Gly Thr Asp Asp Leu Gly Tyr Gly Tyr Ser Ala Ala Thr Ala
       315                 320                 325
   Leu Ser Ala Ser Gln Ala Leu Leu Asn Ala Asp Thr Tyr Ala Leu Phe
   330                 335                 340                 345
   Ala Asn Ala Val Asn Leu Asn Cys Thr His Val Leu Ala Ile Gly Asn
                   350                 355                 360
   Leu Glu Gly Val Leu Pro Ala Arg Cys Ile Gly Lys Leu Ser Glu Tyr
               365                 370                 375
   Met Ile Gly Ala Asp Gly Ile Lys Val Tyr Thr Met Arg Tyr Asn Gly
           380                 385                 390
   Ala Ile His Leu
       395